# EUROPEAN PATENT APPLICATION

(11) **EP 1 510 576 A1**
(43) Date of publication of application: **02.03.2005**
(21) Application number: 03752922.9
(22) Date of filing: 20.05.2003
(51) Int. Cl.: C12N 15/09, C12Q 1/68

(54) **METHODS OF IDENTIFYING NUCLEIC ACIDS**

(30) Priority: 21.05.2002 JP 2002182177
(71) Applicant: Adgene Co., Ltd., Kumagaya-shi, Saitama 360-0831 (JP)
(72) Inventor: OSHIMA, Joji, Kumagaya-shi, Saitama 360-0831 (JP); NEMOTO, Ken, Tokyo 157-0073 (JP)
(74) Representative: Bassil, Nicholas Charles
(86) International application number: PCT/JP2003/006275
(87) International publication number: WO 2003/097828

(57) **Abstract**

Test nucleic acids are identified by the steps of: synthesizing multiple nucleic acids that comprise nucleotide sequences complementary to different regions of a test nucleic acid; and comparing the dissociation curve of a mixture of the synthesized nucleic acids. The dissociation curve of a mixture of nucleic acids shows a waveform pattern that is unique to the test nucleic acid. Many types of nucleic acids can be efficiently identified using simple reactions. The multiple types of nucleic acids that are necessary for analysis can be easily synthesized by using a primer complex that anneals to multiple regions of the test nucleic acid.

SEQUENCE LISTING

<110> Adgene Co., Ltd.

<120> METHODS FOR IDENTIFYING NUCLEIC ACIDS

<130> ADG-A0301Y1P

<150> JP 2002-143708
<151> 2002-05-20

<150> JP 2002-182177
<151> 2002-05-21

<160> 10

<170> PatentIn version 3.1

<210> 1
<211> 10
<212> DNA
<213> Artificial

<220>
<223> an artificially synthesized primer sequence

<400> 1
agctcgtaaa 10

<210> 2
<211> 10
<212> DNA
<213> Artificial

<220>
<223> an artificially synthesized primer sequence

<400> 2
agttcgtaat 10

<210> 3
<211> 10
<212> DNA
<213> Artificial

<220>
<223> an artificially synthesized primer sequence

<400> 3
aggttgtaaa 10

<210> 4

<211> 10

<212> DNA

<213> Artificial

<220>

<223> an artificially synthesized primer sequence

<400> 4

agttcgtaaa 10

<210> 5

<211> 10

<212> DNA

<213> Artificial

<220>

<223> an artificially synthesized primer sequence

<400> 5

agbtygtaaw 10

<210> 6

<211> 19

<212> DNA

<213> Artificial

<220>

<223> an artificially synthesized primer sequence

<220>

<221> misc_feature

<222> (1)..(2)

<223> n indicates g, a, c or t.

<400> 6

nnvhdbssga tccaaccgc 19

<210> 7

<211> 18

<212> DNA

<213> Artificial

<220>

<223> an artificially synthesized primer sequence

<400> 7

cagcagccgc ggtaatac 18

<210> 8

<211> 18

<212> DNA

<213> Artificial

<220>

<223> an artificially synthesized primer sequence

<400> 8

acgacacgag ctgacgac 18

<210> 9

<211> 11

<212> DNA

<213> Artificial

<220>

<223> an artificially synthesized primer sequence

<400> 9

gatccaaccg c 11

<210> 10

<211> 11

<212> DNA

<213> Artificial

<220>

<223> an artificially synthesized primer sequence

<400> 10

ggaaggtggg g 11

## Description

### Technical Field

The present invention relates to methods for identifying nucleic acids.

### Background Art

The polymerase chain reaction (PCR) method is widely used as a method for amplifying nucleic acids. The PCR method exponentially amplifies nucleic acids, by repeating a reaction that uses the action of DNA polymerase to synthesize a complementary strand from the 3' end of a primer. The primers are oligonucleotides that comprise a nucleotide sequence complementary to the 3'-end nucleotide sequence of the nucleic acid to be amplified. By providing primers for each of the sense and antisense strands, the newly synthesized nucleic acids function as new templates for the next step of the reaction. As a result, exponential amplification is accomplished.

In PCR, primers are highly specific to the template DNA, and recognize only a specific site within the entire DNA. Thus, only one type of PCR product is amplified. PCR is described in detail in Examined Published Japanese Patent Application No. (JP-B) Hei 4-67957.

JP-B Hei 4-67957 discloses a technique for amplifying and detecting a nucleic acid sequence present in only a small amount, with the purpose of applying it to the genetic diagnosis of genetic diseases, cancerous diseases, infectious diseases, or such. This technique uses primers complementary to the nucleotide sequence of the target nucleic acid to detect the presence of a specific sequence in the nucleic acids.

Methods that use PCR to detect or identify nucleic acids use the amount of PCR amplification product as an indicator. For example, amplification of a given length of nucleic acid implies the presence of a detection target. Amplification products can be easily detected by techniques such as electrophoresis. However, since electrophoretic separation techniques are time- and labor-consuming, they are inconvenient for the quick analysis of a large quantity of samples. Accordingly, several methods for rapidly detecting PCR amplification products have been put into practical use.

For example, methods that use an intercalator to optically detect the production of a double-stranded nucleic acid are well known. Intercalators are dyes that emit fluorescence when they bind specifically to double-stranded nucleic acids. Since PCR amplification products form double strands, adding an intercalator to the reaction system enables detection of the amount of amplification product, using changes in fluorescence intensity. Ethidium bromide, sybergreen, or such may also be used as intercalators. An apparatus that detects mutations in nucleic acids by using an intercalator to compare changes in melting temperature (Tm) values is also known (Unexamined Published Japanese Patent Application No. (JP-A) Hei 7-31500).

The progress of a PCR reaction can be monitored using an intercalator. Since this method enables monitoring while a reaction is in progress, it is called 'real-time PCR'. However, detection methods using an intercalator use double strand formation as an indicator, and thus they sometimes cannot detect slight differences in the nucleotide sequences of template nucleic acids. In other words, the specificity of nucleic acid detection methods using intercalators depends on the PCR specificity.

PCR can be used to identify specific nucleotides in template nucleic acid sequences. Primers are necessary for the template-dependent complementary strand synthesis that constitutes the PCR method. Primers are oligonucleotides that comprise a nucleotide sequence complementary to a template nucleic acid. Complementary strand synthesis progresses in a 5' to 3' direction, from the 3' end of the primers annealed to the template nucleic acid. The nucleotides at the 3' end of the primer are important in complementary strand synthesis. More specifically, complementarity of the 3'-end region of a primer with the template nucleic acid greatly influences the efficiency of the complementary strand synthesis reaction.

Thus, complementary strand synthesis is markedly inhibited when the 3' end of a primer is not complementary to a template nucleic acid. Specific nucleotides in the template nucleic acid can be identified using this characteristic. Specifically, the 3' end of a primer is designed to correspond to positions complementary to the nucleotides to be identified in the template nucleic acid. If amplification products are produced using this primer, the nucleotides to be identified are shown to be complementary to the 3' end of the primer. However, this method cannot identify nucleotides in the template nucleic acid that exist in places other than where the primer anneals.

Methods that utilize PCR to detect undiscovered differences in the nucleotides of amplification products are known. For example, in PCR-SSCP, differences in the three-dimensional structure of the PCR amplification products are detected using electrophoresis. Even if DNAs are amplified using the same primer set, their nucleotide sequences are predicted to be different when differences are observed in their three-dimensional structure.

Similarly, a method is known for detecting differences in the nucleotides of template nucleic acids by comparing the restriction enzyme cleavage patterns of PCR products. This method is called PCR-RFLP. Both PCR-SSCP and PCR-RFLP require PCR amplification products to be separated by electrophoresis.

As a method that allows differentiation of the nucleotide sequences of PCR amplification products, the present inventors have completed the development of, and submitted a patent application (JP-A 2002-325581) for, a method that uses dissociation curves as indicators. As temperature increases, PCR amplification products eventually dissociate into single strands. Dissociation to single-stranded nucleic acids is known to occur rapidly at a certain temperature. The temperature at which a double strand rapidly dissociates is called melting temperature (Tm). Tm is determined by the nucleotides comprised in the nucleic acid, and the reaction solution components. Therefore, in reaction solutions with the same composition, Tm depends on the nucleotide sequence that makes up the nucleic acid. The principle of JP-A 2002-325581 is to focus on this characteristic, to detect differences in the nucleotide sequences of PCR amplification products using differences in melting temperatures. Melting temperatures can be easily measured using an intercalator. More specifically, JP-A 2002-325581 allows determination of differences in the nucleotide sequences of PCR amplification products, without depending on complicated techniques such as electrophoresis.

However, in JP-A 2002-325581, nucleic acids are synthesized by PCR, and hence the amplification products lack diversity. In principle, amplification by one set of primers only produces one type of product. This characteristic indicates that PCR is highly specific, but also means that it is difficult to distinguish between each of many structurally similar nucleic acids.

For example, if using one set of primers to distinguish between each of three nucleic acids, A, B, and C, which have very similar structures, the primers must be designed such that nucleotides unique to each of the nucleic acids are included in a region amplified by the same primer set. As the types of nucleic acid increase, it becomes increasingly difficult to amplify the unique regions of all nucleic acids using only one set of primers.

Using many primer sets to perform a similar analysis on multiple regions increases the possibility of being able to distinguish a large variety of nucleic acids. However, using many sets of primers leads to an increase in the number of reactions. More specifically, this can sacrifice the speed and economical efficiency of analysis. Furthermore, as the number of reactions increases, the amount of sample consumed also increases.

### Disclosure of the Invention

An objective of the present invention is to provide methods for easily detecting differences in the nucleotide sequences of nucleic acids, and primers used for this purpose.

In PCR, using multiple primers can sacrifice speed and economic efficiency. Accordingly, the present inventors carried out exhaustive studies on methods that can show differences in the nucleotide sequences of template nucleic acids, without using PCR. As a result, the present inventors completed the present invention by synthesizing complementary strands for multiple regions of nucleic acids to be identified. They also showed that differences between nucleic acids can be detected by comparing the dissociation curve waveform patterns of mixtures of the synthesized complementary strands. Furthermore, the present inventors succeeded in providing primers useful for synthesizing multiple regions of nucleic acids to be identified. By using the primers designed by the present inventors, it is easy to obtain mixtures of complementary strand synthesis products necessary for the identification methods of this invention. More specifically, the present invention relates to methods for identifying nucleic acids, as well as primers useful for synthesizing nucleic acids, as follows:
[1] A method for identifying a nucleic acid, wherein the method comprises the steps of:
   (1) synthesizing a nucleic acid comprising a nucleotide sequence complementary to multiple regions of a test nucleic acid;
   (2) obtaining dissociation curves for a mixture of the nucleic acid synthesized in step (1); and
   (3) comparing the waveform patterns of the dissociation curves and identifying nucleic acids comprising the same waveform pattern to have the same nucleotide sequence.
[2] The method of [1], wherein step (1) of synthesizing a nucleic acid comprising a nucleotide sequence complementary to multiple regions of a test nucleic acid, comprises the step of synthesizing a complementary strand by annealing one or more types of primer comprising a nucleotide sequence complementary to multiple regions of the test nucleic acid.
[3] The method of [2], which comprises the step of synthesizing the complementary strand by annealing the primers in the presence of a denaturant and/or salt.
[4] The method of [3], wherein the denaturant is selected from the group consisting of nonionic surfactants, anionic surfactants, and detergents.
[5] The method of [4], wherein the nonionic surfactant is any one selected from the group consisting of a polyoxyethylene ether of glycerol ester, a polyoxyethylene ether of sorbitan ester, and a polyoxyethylene ether of sorbitol ester.
[6] The method of [4], wherein the detergent is any compound selected from the group consisting of dodecyl sulfate, lauroylsarcosine salt, laurylate, and mercaptoacetate.
[7] The method of [3], wherein the salt is any compound selected from the group consisting of Na₂SO₄, Na₂SO₃, NaH₂PO₄, and NaHCO₃.
[8] The method of [2], wherein the primers comprise one type of oligonucleotide that can anneal to multiple regions of the test nucleic acid.
[9] The method of [2], wherein the primers comprise two or more types of oligonucleotides that can anneal to multiple regions of the test nucleic acid.
[10] The method of [9], wherein the nucleotide sequences of the multiple regions are partially identical.
[11] The method of [10], wherein the nucleotide sequences of the primers are partially different, and wherein the different nucleotides are at any position on the primer nucleotide sequence.
[12] The method of [10], wherein the nucleotide sequences of the primers are partially different, and wherein the different nucleotides are localized to the 5'-side of the primer nucleotide sequence.
[13] The method of [12], wherein the primers constitute a primer complex for producing waveforms, wherein the primer complex comprises:
   a specific primer, which comprises a nucleotide sequence complementary to a target region of a template nucleic acid; and
   at least one type of ambiguous primer, which comprises the following specific region and ambiguous region:
      a specific region, which comprises the 3' end of the oligonucleotide and consists of a nucleotide sequence complementary to the target region; and
      an ambiguous region, which is positioned to the 5' side of the specific region, and comprises a nucleotide sequence wherein a nucleotide comprised in the nucleotide sequence complementary to the target region is substituted with a nucleotide other than the nucleotide.
[14] A method for identifying a nucleic acid, wherein the method comprises the steps of:
   selecting multiple regions as target regions in a template nucleic acid; and
   performing the method of [13] on a single test nucleic acid using the multiple target regions as objects of analysis.
[15] The method of [13], which comprises the step of:
   annealing the primer complex to the template nucleic acid at a temperature that is 20°C to 40°C lower than the melting temperature of the specific primer.
[16] The method of [13], wherein multiple cycles of the primer complex annealing step and the complementary strand synthesis step are performed.
[17] The method of [1] , wherein the test nucleic acid is single stranded or double stranded.
[18] The method of [1] , wherein the test nucleic acid is a DNA or RNA.
[19] The method of [1], wherein the test nucleic acid is a genomic DNA, and wherein step (1) comprises the step of synthesizing at least one region whose nucleotide sequence in a cell to be identified differs from that in an another cell.
[20] The method of [19], wherein the nucleotide sequences of the primers for synthesizing multiple regions are at least partially identical.
[21] The method of [19], wherein the test nucleic acid is a genomic DNA of a microorganism, and wherein step (1) comprises the step of synthesizing at least one region whose nucleotide sequence in the microorganism to be identified differs from that in an another microorganism.
[22] The method of [19], wherein the test nucleic acid is a genomic DNA of a eukaryotic cell, and wherein step (1) comprises the step of synthesizing a region comprising a group of genes in which a nucleotide sequence is conserved.
[23] The method of [22], wherein the method comprises the step of synthesizing multiple regions using a primer comprising a nucleotide sequence complementary to a nucleotide sequence that is conserved among the genes.
[24] A method for producing dissociation curve waveform patterns as a reference for identifying a nucleic acid, wherein the method comprises the steps of:
   (1) synthesizing nucleic acids, which comprise nucleotide sequences complementary to multiple regions of a standard nucleic acid sample, as test nucleic acids; and
   (2) obtaining dissociation curves for the mixture of nucleic acids synthesized in step (1).
[25] A method for producing a reference dissociation curve waveform pattern for multiple types of standard nucleic acid samples by using the method of [24].
[26] The method of [25], wherein the method comprises the step of obtaining a dissociation curve for multiple types of standard nucleic acid samples that are synthesized by a common primer complex.
[27] A reference dissociation curve waveform pattern database that comprises multiple reference dissociation curve waveform patterns, obtained by the method of [24].
[28] A primer complex for waveform production, which comprises a mixture of one or more types of primers whose nucleotide sequences are complementary to multiple regions in a test nucleic acid.
[29] The primer complex of [28], wherein the primer is one type of oligonucleotide that can anneal to multiple regions in the test nucleic acid.
[30] The primer complex of [28], wherein the primers are two or more types of oligonucleotides that can anneal to multiple regions in the test nucleic acid.
[31] The primer complex of [30], wherein the nucleotide sequences of the multiple regions are partially identical.
[32] The primer complex of [30], wherein the nucleotide sequences of the primers are partially different, and wherein the different nucleotides are at any position in the primer nucleotide sequence.
[33] The primer complex of [30], wherein the nucleotide sequences of the primers are partially different, and wherein the different nucleotides are localized to the 5' side of the primer nucleotide sequence.
[34] The primer complex of [33], that comprises:
   a specific primer, which comprises a nucleotide sequence complementary to a target region of a template nucleic acid; and
   at least one type of ambiguous primer, which comprises the following specific region and ambiguous region:
      a specific region, which comprises the 3' end of a primer and consists of a nucleotide sequence complementary to the target region; and
      an ambiguous region, which is positioned to the 5' side of the specific region, and comprises a nucleotide sequence wherein a nucleotide comprised in the nucleotide sequence complementary to the target region is substituted with a nucleotide other than the nucleotide.
[35] The primer complex of [34], wherein the gc content in the specific region of each primer is 50% or more.
[36] The primer complex of [34], wherein the primer complex is used for waveform production and wherein the variety of the substituted nucleotide in the ambiguous region of the ambiguous primer increases from the 3' side to the 5' side.
[37] The primer complex of [34], wherein the nucleotide sequence of the ambiguous region comprises the following three regions, and wherein the primer complex comprises ambiguous primer complexes having all combinations of substituted nucleotide sequences that constitute each of the three regions,
   (1) an N region constituting the 5'-terminus of the ambiguous region, in which each of the nucleotides of its nucleotide sequence are substituted with random three types of nucleotides other than the nucleotide complementary to a target region nucleotide, wherein the three types of nucleotides are selected from adenine, cytosine, guanine, and thymine,
   (2) a 3 ambiguous region, positioned to the 3'-side of the N region, in which each of the nucleotides of its nucleotide sequence are substituted with random two types of nucleotides other than the nucleotide complementary to a target region nucleotide, wherein the three types of nucleotides are selected from adenine, cytosine, guanine, and thymine, and
   (3) a 2 ambiguous region, positioned to the 3'-side of the 3 ambiguous region, in which each of the nucleotides of its nucleotide sequence are substituted with a random type of nucleotide other than the nucleotide complementary to a target region nucleotide, wherein the random type of nucleotide is selected from adenine, cytosine, guanine, and thymine.
[38] The primer complex of [37], wherein the number of nucleotides of the N region of the primer is two to four.
[39] The primer complex of [37], wherein the ratio of the number of nucleotides of the N region: 3 ambiguous region: 2 ambiguous region of the primer is 1:2:1.
[40] The primer complex of [34], wherein the number of nucleotides of an ambiguous region of an ambiguous primer of the primer complex is 10% to 80% of the number of nucleotides of the primer.
[41] The primer complex of [34], wherein the total number of nucleotides in the specific region and ambiguous region of the ambiguous primers of the primer complex is ten to 30 nucleotides.
[42] A method for producing a primer complex for waveform production, wherein the complex comprises:
   a specific primer, which comprises a nucleotide sequence complementary to a target region of a template nucleic acid; and
   at least one type of ambiguous primer, which comprise the following specific region and ambiguous region:
      a specific region, which comprises the 3' end of a primer and consists of a nucleotide sequence complementary to the target region; and
      an ambiguous region, which is positioned to the 5' side of the specific region, and comprises a nucleotide sequence wherein the nucleotide comprised in the nucleotide sequence complementary to the target region is substituted with a nucleotide other than the nucleotide; and
   wherein the method comprises the steps of:
   a) synthesizing the specific region; and
   b) synthesizing the ambiguous region by binding a nucleotide of a nucleotide sequence complementary to the target region, to a mixture of random nucleotides other than said nucleotide, selected from adenine, cytosine, guanine, and thymine.
[43] The method of [42], wherein the number of random nucleotides increases from one to three starting from the 3' side to the 5' side of the ambiguous region.
[44] A kit for identifying a nucleic acid, wherein the kit comprises the components of:
   (1) a primer complex for waveform production, that comprises a mixture of one or more types of primers comprising nucleotide sequences complementary to multiple regions of a test nucleic acid;
   (2) a DNApolymerase that catalyzes template-specific synthesis of a complementary strand; and
   (3) a substrate for complementary strand synthesis.
[45] The kit of [44], wherein the primers are one type of oligonucleotide that can anneal to multiple regions of the test nucleic acid.
[46] The kit of [44], wherein the primers are two or more types of oligonucleotides that can anneal to multiple regions of the test nucleic acid.
[47] The kit of [46], wherein the nucleotide sequences of multiple regions are partially identical.
[48] The kit of [47], wherein the nucleotide sequences of the primers are partially different, and wherein the different nucleotides are at any position in the primer nucleotide sequence.
[49] The kit of [47], wherein the nucleotide sequences of the primers are partially different, and wherein the different nucleotides are localized to the 5' side of the nucleotide sequences of the primers.
[50] The kit of [49], wherein the primer complex comprises a complex of:
   a specific primer, which comprises a nucleotide sequence complementary to the target region of a template nucleic acid; and
   at least one type of ambiguous primer, which comprises the following specific region and ambiguous region:
      a specific region, which comprises the 3' end of a primer and consists of a nucleotide sequence complementary to the target region; and
      an ambiguous region, which is positioned to the 5' side of the specific region, and comprises a nucleotide sequence wherein a nucleotide comprised in the nucleotide sequence complementary to the target region is substituted with a nucleotide other than the nucleotide.
[51] The kit of [49], which comprises multiple target regions.
[52] The kit of [50], wherein the primer complex for waveform production for multiple regions is pre-loaded into individual reaction vessels.
[53] The kit of [49], which further comprises the dissociation curve waveform patterns of a positive control and/or nucleic acid to be identified.
[54] The kit of [44], which further comprises a denaturant and/or salt.
[55] The kit of [54], wherein the denaturant is selected from the group consisting of nonionic surfactants, anionic surfactants, and washing agents.
[56] The kit of [55], wherein the nonionic surfactant is selected from the group consisting of a polyoxyethylene ether of glycerol ester, a polyoxyethylene ether of sorbitan ester, and a polyoxyethylene ether of sorbitol ester.
[57] The kit of [55], wherein the detergent is any compound selected from the group consisting of dodecyl sulfate, lauroylsarcosine salt, laurylate, and mercaptoacetate.
[58] The kit of [54], wherein the salt is any compound selected from the group consisting of Na₂SO₄, Na₂SO₃, NaH₂PO₄, and NaHCO₃.

The present invention also provides methods for identifying nucleic acids, where the methods comprise the steps of:
(1) synthesizing a nucleic acid comprising a nucleotide sequence complementary to multiple regions of a test nucleic acid;
(2) obtaining dissociation curves for a mixture of the nucleic acid synthesized in step (1); and
(3) comparing the waveform patterns of the dissociation curves and identifying nucleic acids comprising the same waveform pattern to have the same nucleotide sequence.

The present invention can identify any nucleic acid used as a test nucleic acid. Nucleic acids comprise DNAs, RNAs, and their derivatives. DNA and RNA sources are not limited. Normally, nucleic acid samples can be obtained from biological materials. Specifically, the biological materials can be various cells, bloods and body fluids, plant and animal tissues, and such. In addition, nucleic acids obtainable by using various artificial methods to synthesize all or a portion of a nucleic acid from such materials, and DNAs obtainable by reverse transcribing RNA materials, can also be used as the materials. DNAs and RNAs may also be derived from viruses and vectors. Nucleic acids artificially synthesized as information vehicles or operational elements may also be used.

Examples of DNA or RNA derivatives include:
- DNAs, RNAs, or such that are synthesized with a nucleotide derivative as the constitutional unit; and
- DNAs, RNAs, or such that have been modified by other molecules.

In the present invention, "multiple regions" in the above-described phrase "synthesis of a nucleic acid comprising nucleotide sequences complementary to multiple regions of a nucleic acid to be identified", are selected from multiple regions in the nucleotide sequence of a nucleic acid to be identified. In the cells of animals and plants, one genome set is maintained in multiple chromosomes. In such cells, multiple regions are selected by using all of the multiple nucleic acids constituting one genome set as a target. In other words, multiple sites can be selected from some of the chromosomes. Alternatively, multiple regions can be selected from the multiple chromosomes that make up a single genome set. In the present invention, the term "multiple" refers to at least two or more regions, such as two to 150, or preferably five to 80 regions. Increasing the number of regions to be synthesized increases the sensitivity of the identification methods of this invention. In the present invention, increased sensitivity means that the amount of nucleic acid required for identification can be reduced. The regions to be synthesized are usually designed so that they do not overlap. However, overlap between regions is acceptable when it does not interfere with complementary strand synthesis.

The multiple regions preferably comprise regions where differences in nucleotide sequence can be anticipated between the nucleic acids being compared. When various types of nucleic acids are subject to comparison, the nucleotide sequences of the regions in each nucleic acid to be synthesized are preferably designed so that at least one of the regions comprises a difference when compared to the other nucleic acids. Not all of the multiple regions need to comprise a difference in nucleotide sequence. Take, for example, the synthesis of three regions, "a" to "c", using three nucleic acids, X, Y, and Z. Differences in the nucleotide sequence of each region are indicated by"'". In the example below, nucleic acid Y comprises nucleotide sequence region "b'", which is different from the other nucleic acids. Similarly, nucleic acid Z comprises "c'". The three nucleic acids can be distinguished by synthesizing these regions.
Nucleic acid X: [a]-[b]-[c]
Nucleic acid Y: [a]-[b']-[c]
Nucleic acid Z: [a]-[b]-[c']

In the nucleic acid identification methods of this invention, the aforementioned multiple regions preferably retrieve specific nucleotide sequence information with as much variety as possible from the test nucleic acids. Specifically, it is preferable to synthesize as many as possible of those portions in which nucleotide sequences differ between test nucleic acids. Therefore, in the above-described example of nucleic acids X to Z, for example, it is preferable that the nucleotide sequences are mutually different in as many regions of "a" to "c" as possible.

Genomic nucleotide sequence information has already been elucidated for a number of biological species with genomes of limited size, such as microorganisms and viruses. In such biological species, the nucleotide sequence regions that differ between the biological species subject to comparison and other species can be selected in advance. Even when genomic nucleotide sequence information is insufficient, regions where the nucleotide sequences differ can be selected based on information about genomes and genes that have already been identified.

Multiple regions are preferably synthesized by a reproducible method. In the present invention, "reproducibility" of complementary strand synthesis means that the same region can be used as a template for complementary strand synthesis. There may be cases where the synthesized products cannot be guaranteed to be of uniform length, depending on the principle of the complementary strand synthesis. In the present invention, if the same region is used as a template, variation in the length of the synthesized products is acceptable. Reproducible complementary strand synthesis of this invention may be also defined such that a certain primer specifically initiates complementary strand synthesis. Therefore, the complementary strand synthesis of this invention can be distinguished from complementary strand synthesis by random primers.

When synthesizing multiple regions for multiple nucleic acids, it goes without saying that each nucleic acid must be synthesized under the same conditions. Therefore, when synthesizing multiple regions by complementary strand synthesis using primers and DNA polymerases, it is preferable to maintain specific primer hybridization.

The nucleic acid synthesis methods are not particularly limited, as long as they are template-dependent synthesis methods. When the test nucleic acid is double-stranded, multiple regions can be synthesized using the sense or antisense strand as the template. All of the multiple regions may be synthesized by using the sense or antisense strand as the template, or by using both strands as templates.

Next, a method for obtaining elongation products for the present invention is described. As described above, a representative method for obtaining elongation products is the use of complementary strand synthesis using DNA polymerase. In this invention, primers that can anneal to multiple regions of a test nucleic acid are used to synthesize multiple regions of a test nucleic acid. In the present invention, "elongation products" refers to elongation products synthesized by complementary strand synthesis using primers that can anneal to multiple regions of a test nucleic acid. Elongation products are a group of polynucleotides that have been synthesized using multiple regions of a test nucleic acid as templates. Therefore, the nucleotide sequences of each polynucleotide strand of the elongation products differ from each other. However, repeating complementary strand synthesis produces many molecules of polynucleotide strands comprising the same nucleotide sequence.

Furthermore, when the template is a test nucleic acid comprising the same nucleotide sequence in different regions, elongation products comprising the same nucleotide sequence may be produced. However, in the present invention it is preferable to obtain various waveform patterns of dissociation curves. Thus the nucleotide sequences of elongation products are preferably designed to be as different to each other as possible.

In this invention, primers that allow complementary strand synthesis in multiple regions by annealing to multiple regions of a test nucleic acid are called "primers for waveform production". The primers for waveform production of this invention are used for complementary strand synthesis of multiple regions of a test nucleic acid. As a result of complementary strand synthesis, they yield a mixture of elongation products, comprising different nucleotide sequences. In this invention, a mixture of different elongation products preferably refers to a mixture of polynucleotides that yield multiple Tm's when the dissociation curves are analyzed.

The primers for waveform production of this invention can also be defined as follows: The primers for waveform production of this invention are oligonucleotides comprising nucleotide sequences complementary to specific nucleotide sequence regions at multiple regions on a test nucleic acid. The primers can initiate template-dependent complementary strand synthesis by DNA polymerase, in multiple regions of the test nucleic acid, under identical conditions. At least a part of the nucleotide sequence of a primer for waveform production comprises a nucleotide sequence complementary to a specific region, as mentioned above. A nucleotide sequence complementary to an above-mentioned specific region can be positioned at an arbitrary region of an oligonucleotide. More specifically, it can be positioned, for example, in a region containing the 3' end of the oligonucleotide, a region in the middle that does not contain an end, and a region containing the 5' end.

Examples of methods for designing primers that can anneal to multiple regions of a test nucleic acid are described below. By designing primers based on the following concepts, the primers can be synthesized more easily than by just mixing the primers necessary for complementary strand synthesis of multiple regions.

### One type of primer that anneals to multiple regions:

If an appropriate nucleotide sequence is selected, even a single primer can anneal to multiple regions of a test nucleic acid. For sequence-specific annealing, an oligonucleotide of 20 to 50 nucleotides is usually used as a primer. Under stringent conditions, there is a very low possibility that an oligonucleotide of such length will anneal to multiple regions.

However, when an oligonucleotide of fewer nucleotides is used, the possibility that it will anneal at multiple regions is increased. For example, there is a 1/64 (4³) to 1/65536 (4⁸) probability that a region exists to which an oligonucleotide of three to eight nucleotides may anneal. That is, a short primer can enable complementary strand synthesis of multiple regions, even when used alone. When using such a short oligonucleotide as a primer, it is preferable to do so under the conditions of increased primer concentration, lowered annealing temperature, and such.

One such type of primer that can anneal to multiple regions can be designed by searching the nucleotide sequence information of a subject genome for short nucleotide sequences of three to ten nucleotides that frequently appear in the target genome, judging top candidates by their order of the frequency of appearance. The genomic region over which frequency of appearance is tallied may be the information for the entire nucleotide sequence, or a limited region of particular interest.

### Two or more types of primers that anneal to multiple regions:

If the nucleotide sequence of a template nucleic acid is known, it is easy to design multiple primers that anneal to each of the regions in order to synthesize multiple regions. However, in some cases in the present invention, complementary strand synthesis is necessary in more regions. As the regions to be synthesized increase, the number of necessary primers also increases. On the other hand, there is an upper limit to the amount of oligonucleotides that can be added to a reaction system. Furthermore, it is not economical to synthesize a large variety of oligonucleotides. Therefore, the present inventors investigated a method that can accomplish the complementary strand synthesis of many regions using the least number of primer types. As a result, they found that primers designed as shown below are effective.

Firstly, the regions to be synthesized are selected to maximize nucleotide sequence commonality between primers. For example, in the following example, two regions for annealing the primer are selected. These nucleotide sequences were selected from the genomes of two types of acid-fast bacteria: A (*Mycobacterium bovis*) and B (*Mycobacterium kansasii*)*.* Although positioned differently on the genome, their nucleotide sequences are highly similar (matching nucleotides are shown in capital letters).

Acid-fast bacterium A: AGcTcGTAAa (SEQ ID NO: 1)

Acid-fast bacterium A: AGtTcGTAAt (SEQ ID NO: 2)

Acid-fast bacterium B: AGgTtGTAAa (SEQ ID NO: 3)

Acid-fast bacterium B: AGtTcGTAAa (SEQ ID NO: 4)

In this example, the 3rd, 5th, and 10th nucleotides are different, and the remaining nucleotides match. Expressing these different nucleotides in ambiguity codes yields agBtYgtaaW (SEQ ID NO: 5). In the present invention, the term "ambiguity code" refers to a code for assigning multiple types of nucleotides to a certain position. In the present invention, oligonucleotides represented by an ambiguity code mean a mixture that contains all combinations of nucleotides represented by an ambiguity code at the ambiguity code positions. The ambiguity codes used herein correspond to the following nucleotides:
B: c, g, and t Y: c and t W: a and t

More specifically, in this example, by selecting nucleotide sequences that are highly similar (agBtYgtaaW), it becomes possible to synthesize complementary strands at two regions for one bacterial strain. In this invention, primers comprising nucleotide sequences that can be represented by ambiguity codes are called "ambiguou s primers".

Usually, the synthesis of strands complementary to four regions requires four types of primers annealing to each region. However, in the examples herein, one type of ambiguous primer enabled the synthesis of strands complementary to four regions. Ambiguous primers are a group of oligonucleotides comprising nucleotide sequences that differ from each other. However, in terms of manipulation, they are synthesized by operations similar to that for single oligonucleotides, and they can also be used like single oligonucleotides during complementary strand synthesis.

Furthermore, based on this strategy, one type of ambiguous primer can be designed to be common to a number of specific regions of interest within a genome. In the case of the acid-fast bacteria A and B, the present invention achieves the objective of bacterial strain identification by selecting nucleotide sequences that show the most remarkable variation between species A and B, and in addition, the present invention also provides a means for simultaneously obtaining information relating to a completely different region of interest, such as a region related to drug resistance. More specifically, a single type of ambiguous primer, which can simultaneously yield more than one type of completely independent information, can be designed by selecting candidate nucleotide sequences that each have a high frequency of appearance in multiple regions of interest, and further selecting those nucleotide sequences that show higher similarities from among the top candidate nucleotide sequences.

Generally, degenerate primers are often used when a portion of the nucleotide sequence of a template nucleic acid is unknown. The nucleotide sequences of degenerate primers are denoted in the same way as for the ambiguous primers of this invention. However, ambiguous primers are used to synthesize multiple regions comprising different nucleotide sequences. On the other hand, degenerate primers used for PCR cloning and such are usually used to synthesize one type of nucleic acid for each type of template. When used for cloning (that is, to isolate a gene), primers that produce a variety of synthesis products are undesirable. As described above, ambiguous primers and degenerate primers are designed for completely different purposes. On the other hand, degenerate primers can be utilized as the waveform production primers of this invention.

In the examples shown above, regions that can minimize the number of primer types were selected from the nucleotide sequence of a test nucleic acid. In other words, this design method depends on the test nucleic acid. Thus, this method may be difficult to apply, depending on the nucleotide sequence of the test nucleic acid and that of the nucleic acid for comparison. In contrast, the methods described below enable multiple primers to be designed relatively easily, without being affected by the nucleotide sequences of the test nucleic acid and the nucleic acid being compared.

Ambiguity codes were positioned in ambiguous primers, according to the nucleotide sequence of template nucleic acids. In contrast, primer complexes for waveform formation, which can anneal to multiple regions of a test nucleic acid, can be obtained by placing ambiguity codes at the 5' side of the primers. More specifically, the present invention relates to primer complexes for waveform production, which comprise:
a specific primer, which comprises a nucleotide sequence complementary to a target region of a template nucleic acid; and
at least one type of ambiguous primer, which comprises the following specific region and ambiguous region:
   a specific region, which comprises the 3' end of the oligonucleotide and consists of a nucleotide sequence complementary to the target region; and
   an ambiguous region, which is positioned to the 5' side of the specific region, and comprises a nucleotide sequence wherein a nucleotide comprised in the nucleotide sequence complementary to the target region is substituted with a nucleotide other than the nucleotide.

The specific primers of this invention comprise oligonucleotides that have nucleotide sequences complementary to target regions of template nucleic acids, or derivatives thereof. The derivatives comprise oligonucleotides containing additional nucleotide sequences, or modified oligonucleotides. The oligonucleotides can be modified using fluorescent substances, radioactive substances, or affinity ligands.

The term "target regions" in the present invention refers to the regions to which primers for the complementary strand synthesis of template nucleic acids should anneal. That is, a target region is positioned at the 3' side of the region to be synthesized of the template nucleic acid. As mentioned above, in the methods for identifying nucleic acids of this invention, regions of the template nucleic acid to be synthesized are preferably selected as regions in which a waveform pattern unique to a test nucleic acid is expected to occur. Such regions can be selected by focusing on regions that comprise nucleotide sequences different to the nucleic acids to be compared. On the other hand, in order to synthesize multiple regions using the minimum primer types, for example, highly homologous nucleotide sequences found at different positions can be selected as target regions. However, by using a primer complex for waveform production based on the present invention, primers that can anneal to regions with low homology to the target region can also be provided. As a result, even when a single target region is selected, a primer complex for waveform production can be obtained that makes complementary strand synthesis of multiple regions of a template nucleic acid possible.

In the ambiguous primers, as described below, the 3'-side region, which does not comprise an ambiguity code, is called the specific region; and the 5'-side region, in which ambiguity codes are positioned, is called the ambiguous region. The specific region is made up of a nucleotide sequence identical to the nucleotide sequence that constitutes a region comprising the 3' end of the specific primer. More specifically, the nucleotide sequences comprising the 3'-ends of the ambiguous primer and the specific primer are identical. The number of nucleotides comprised in the specific region is not limited. The length of the specific region can be appropriately determined according to the ambiguous region conditions. For example, the length of the specific region can be selected from 20% to 90% of the length of the entire ambiguous primer.

The ambiguity codes comprised in the ambiguous region may be, for example, a four-nucleotide ambiguity (N) sequence, or necessary ambiguity codes that are appropriately positioned according to the nucleotide sequence of the test nucleic acid, as described in the example of acid-fast bacteria. Furthermore, as described below, the variation of the ambiguous region can be increased stepwise from the 3' side to the 5' side of the primer.

Ambiguous primers designed in this manner anneal to many regions of the test nucleic acid with high reproducibility, yielding products of the complementary strand synthesis of multiple regions. By altering the ambiguous region variation in a stepwise manner, ambiguity is reduced at the 3' side, which influences the reaction specificity of complementary strand synthesis, at the same time diversifying the nucleotide sequence of the primer at the 5' side, which in comparison has hardly any influence on specificity. Such structural characteristics provide a complex of primers that can stably anneal to a variety of nucleotide sequences.

Each of the oligonucleotides that constitute a primer complex is designed to reduce diversity at the 3' side. As a result, they selectively anneal to nucleotide sequences that are complementary to the nucleotide sequence of each oligonucleotide, and initiate complementary strand synthesis. More specifically, complementary strand synthesis can be specifically and stably initiated for each of a wide variety of nucleotide sequences.

The following structures can be indicated as ambiguous primers in which the ambiguity of the ambiguous region is changed stepwise. Specifically, the present invention relates to a primer complex for waveform production which comprise all combinations of substituted nucleotide sequences that constitute each ambiguous region, wherein the nucleotide sequence of the ambiguous region consists of the following three regions:
(1) an N region constituting the 5'-terminus of the ambiguous region, in which each of the nucleotides of its nucleotide sequence are substituted with random three types of nucleotides other than the nucleotide complementary to a target region nucleotide, wherein the three types of nucleotides are selected from adenine, cytosine, guanine, and thymine,
(2) a 3 ambiguous region, positioned to the 3'-side of the N region, in which each of the nucleotides of its nucleotide sequence are substituted with random two types of nucleotides other than the nucleotide complementary to a target region nucleotide, wherein the three types of nucleotides are selected from adenine, cytosine, guanine, and thymine, and
(3) a 2 ambiguous region, positioned to the 3'-side of the 3 ambiguous region, in which each of the nucleotides of its nucleotide sequence are substituted with a random type of nucleotide other than the nucleotide complementary to a target region nucleotide, wherein the random type of nucleotide is selected from adenine, cytosine, guanine, and thymine.

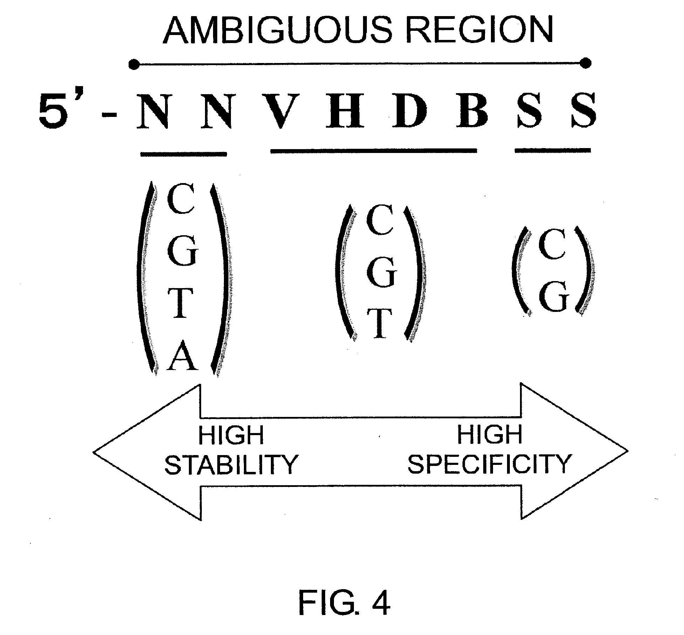

In the ambiguous primer (NNVHDBSS) shown in Fig. 4, the ambiguous region is designed such that the ambiguity increases stepwise in a 5' to 3' direction. More specifically, an N region made up of Ns, N being an ambiguity code indicating any one of adenine (A), cytosine (C), guanine (G), and thymine (T), exists at the 5' end. The N region can also be described as a region in which nucleotides complementary to a target region are substituted with any of the other three nucleotide types.

The 3 ambiguous region (3AR), in which a nucleotide complementary to the target region is substituted with the other two types of nucleotides, is positioned adjacent to the N region. Furthermore, the 2 ambiguous region (2AR), in which a nucleotide complementary to the target region is substituted with another type of nucleotide, is positioned 3' of 3AR. In the example of Fig. 4, two Ns at the 5' end constitute the N region, and the following four nucleotides, V, H, D, and B, constitute 3AR. Furthermore, the two S's at the 3' side constitute 2AR. According to the structure of such an ambiguous region, ambiguity increases in a 3' to 5' direction. In other words, specificity increases from the 5' side to the 3' end in such structures. Table 1 summarizes the ambiguity codes used in the present invention, and the actual corresponding nucleotides.

In the ambiguous primers of this invention, the N region preferably consists of two to four nucleotides. When the number of nucleotides of the N region becomes five or more, the possibility that primers will interfere with each other is increased due to dimers, loops, and such. The stability of the 3AR sequence increases in the order of DHVB, HDVB, VHDB, and HVDB. Accordingly, increasing the proportion of G or C at the 3' side increases stability.

The 2AR sequence must also be designed by taking the nucleotide sequence of the target region into consideration. The ratio of the lengths of the N region, 3AR, and 2AR is preferably 1:2:1, but is not limited thereto.

The number of nucleotides in an ambiguous region of the ambiguous primers constituting a primer complex is preferably 10% to 80% of the number of nucleotides of the primer. Furthermore, the total number of nucleotides in the specific region and ambiguous region of the ambiguous primers constituting the primer complex is preferably ten to 30 nucleotides.

In the primer complexes for waveform production of this invention that comprise ambiguous primers, a target region can be designed based on the nucleotide sequence of at least one region which was set as a target region. A number of target regions can also be selected. In such cases, the types of specific primers increase, and the types of ambiguous primers increase accordingly. When multiple regions are selected as target regions, the primer complexes for waveform production can be used simultaneously or separately for complementary strand synthesis. By using the primer complexes for waveform production, the diversity of the waveform can be improved. More specifically, the ability of the methods of this invention to differentiate nucleic acids can be enhanced.

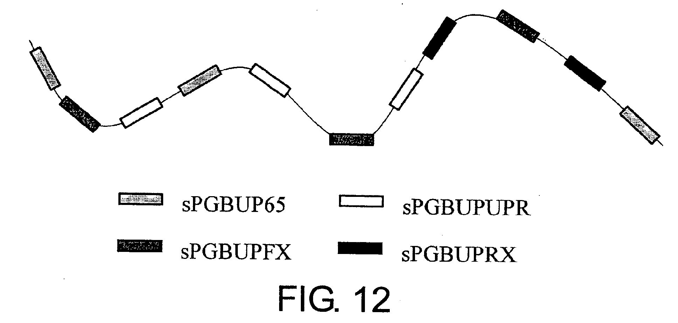

For example, the four types of primer complexes for waveform production, sPGBUP65, sPGBUPUPR, sPGBUPFX, and sPGBUPRX, shown in Fig. 12, are individually designed for different target regions. If each primer complex for waveform production yields 15 different curves, 50,625 types (15 x 15 x 15 x 15) of waveform patterns are obtainable in theory. This indicates that the use of four different primer complexes for waveform production, each able to produce 15 types of waveform pattern, enables the differentiation of 50,000 types or more of nucleic acids by the methods for identifying nucleic acids of this invention.

For complementary strand synthesis, multiple types of the primer complexes for waveform production, which are designed for multiple regions, can be utilized as a mixture of complexes, or separately as individual complexes. Individual use of a primer complex for complementary strand synthesis will clarify the relationship between the waveform pattern and the primer complex. More specifically, for complementary strand synthesis, primer complex A for waveform production, which was designed based on a certain region A; and primer complex B for waveform production, which was designed based on another region B; are preferably used independently, without mixing.

Complementary strand synthesis is initiated by incubating any one of the above-described primers that can initiate complementary strand synthesis in multiple regions, with a template nucleic acid under conditions that allow complementary strand synthesis. Conditions allowing complementary strand synthesis fulfill the following conditions:
the primer can anneal to the template nucleic acid;
the catalytic activity of a template-dependent DNA polymerase is maintained; and
a substrate for complementary strand synthesis is provided.

Conditions in which primers can anneal to template nucleic acids can be appropriately established by one skilled in the art, according to the nucleotide sequence of the primer. For example, as described in Examples, the Tm of a DNA can be calculated by the 2(A+T)+4(G+C) method (Wallace method). Tm can be calculated by other known methods such as the GC% method or Nearest Neighbor method. Furthermore, Tm value is known to decrease on addition of formamide, dimethylsulfoxide (DMSO), or such. On the other hand, Tm is generally known to increase with increasing salt concentration in the reaction solution.

In contrast, the present inventors found that certain types of additives may stabilize the pairing of a primer to a test nucleic acid, and may suppress the inhibition of complementary strand synthesis due to competitive and nonspecific binding of coexisting nucleic acid elongation products to the test nucleic acid. In the present invention, additives used to stabilize the annealing of a primer to a test nucleic acid are called stabilizers. Various denaturants or salts can be used as stabilizers. In the present invention, low temperature conditions are sometimes preferable for complementary strand synthesis that uses primers with short nucleotide sequences, or primers comprising an ambiguous region. More specifically, when using a primer of less than 20 nucleotides, a reaction temperature of 25°C to 50°C or such is desirable. In the present invention, the addition of a stabilizer is effective for such complementary strand synthesis at low temperatures. Surfactants can be denaturants that can be used as stabilizers in this invention.

For example, nonionic surfactants or anionic surfactants may be used as stabilizers. Nonionic surfactants can be categorized into ether type, ether ester type, ester type, and nitrogen-containing type surfactants. Of these nonionic surfactants, ether ester type nonionic surfactants are preferred. More specifically, polyoxyethylene ether of glycerol ester, polyoxyethylene ether of sorbitan ester, and polyoxyethylene ether of sorbitol ester can be the ether ester type nonionic surfactants. Ether ester type nonionic surfactants include polyoxyethyleneglycerol fatty acid ester, castor oil, polyoxyethylenesorbitan fatty acid ester, polyoxyethylenesorbitol fatty acid ester, and poloxyethylene fatty acid alkanolamide sulfate. In the present invention, a particularly favorable ether ester type nonionic surfactant is polyoxyethylenesorbitan fatty acid ester. Examples of commercially available nonionic surfactants that can be utilized as the stabilizers of this invention are shown below:
Polyoxyethylene alkyl ether:
   NIKKOL BL-9EX (Polyoxyethylene(9) Lauryl Ether)
   Brj35 (Polyoxyethylene(23) Lauryl Ether)
Polyoxyethylene alkylphenyl ether:
   TRITON X-114 (Polyoxyethylene(8) Octylphenyl Ether)
   TRITON X-100 (Polyoxyethylene(8) Octylphenyl Ether)
   NP-40 (Polyoxyethylene(9) Octylphenyl Ether)
Polyoxyethylenesorbitan fatty acid ester:
   TWEEN 20 (Polyoxyethylene(20) Sorbitan Monolaurate)
   TWEEN 80 (Polyoxyethylene(20) Sorbitan Monooleate)
   TWEEN 40 (Polyoxyethylene(20) Sorbitan Monopalmitate)
   TWEEN 60 (Polyoxyethylene(20) Sorbitan Monostearate)
   TWEEN 85 (Polyoxyethylene(20) Sorbitan Trioleate)

Anionic surfactants that may be used for the present invention are surfactants such as sulfonates, carboxylates, sulfate esters, or phosphate esters. The following washing agents can also be used as stabilizers. For these detergents, salts other than sodium salts may be used:
Sodium Dodecyl Sulfate (SDS)
Sodium N-Lauroyl Sarcosinate
Sodium Laurate
Sodium Mercaptoacetate

The concentration of a denaturant in the reaction solution can be appropriately adjusted, depending on the nucleotide sequence of the primer complex for waveform formation, and the temperature used for complementary strand synthesis. In general, denaturant is added to achieve 0.01% W/V to 10% W/V, for example, 0.5% W/V to 5% W/V, and more specifically, 1% W/V to 3% W/V.

Furthermore, the present inventors showed that addition of certain types of salts is effective in stable annealing to a test nucleic acid, and in complementary strand synthesis, even when a primer complex for waveform production of this invention consists of relatively short nucleotide sequences. Salts that are added to the reaction solution include Na₂SO₄, Na₂SO₃, NaH₂PO₄, and NaHCO₃. The concentration of these salts in the reaction solution can be appropriately adjusted, according to the nucleotide sequence of the primer complex for waveform production, and the temperature used for complementary strand synthesis. Generally, salts can be added to the reaction solution to 0.005 M to 0.5 M, for example, 0.01 M to 0.1 M, and more specifically, 0.03 M to 0.05 M.

The denaturants or salts given as examples of the stabilizers may be used individually, or in combination with a number of these compounds. For each compound, a different mechanism is considered to increase the stability or specificity of primer annealing. Therefore, the function of a stabilizer is enhanced by adding each of the surfactant, detergent, and salt components.

For primer annealing, a template nucleic acid must be in a state capable of base-pairing, at least in those regions to which the primers anneal. For example, if a template nucleic acid is double stranded, primers can be annealed by first incubating the nucleic acid in denaturing conditions, to convert it into single strands. When the template nucleic acid exists as single strands, primers can anneal to it directly. For example, various RNAs may be used as templates directly.

Any DNA polymerase that comprises the function of synthesizing a nucleotide sequence that is complementary to the nucleotide sequence of a template nucleic acid, using primers annealed to the template nucleic acid, can be used as the template-dependent DNA polymerase of this invention. Examples of currently available DNA polymerases are:
ExTaq, Taq, Z Taq, Pyrobest DNA polymerase (TaKaRa);
Taq DNA Polymerase, Cloned for PCR (Pharmacia);
Hot Star Taq (QIAGEN); and
KOD DNA Polymerase (TOYOBO).

Of these DNA polymerases, Taq DNA polymerase (TaKaRa Ex Taq™ R-PCR Version) and such thermostable DNA polymerases are preferable, since they are highly heat-stable when using reaction systems that regulate DNA synthesis by increasing or decreasing temperature.

These enzymes are utilized under conditions that maintain their catalytic functions. Optimal conditions for each of the enzymes are well known. Factors that influence enzyme activity include temperature, salt concentration, pH, the presence of denaturants and protectants, or various other cofactors required by each of the enzymes. These conditions can be appropriately set by those skilled in the art.

If these conditions are set within the range necessary to maintain enzyme activity, complementary strands can be synthesized using the substrates required for complementary strand synthesis. Ordinarily, four types of deoxynucleotides (dNTPs) are used as substrates for complementary strand synthesis. DNA derivatives may be synthesized using deoxynucleotide derivatives. Deoxynucleotide derivatives modified with fluorescent dyes and affinity ligands may be used as such derivatives.

Operations in nucleic acid synthesis by DNA polymerase are preferably carried out on ice, to prevent undesired reactions, or to protect each of the reaction components and template nucleic acids. In this invention, when using these DNA polymerases, reagent components and samples are also preferably kept under cool conditions until initiation of complementary strand synthesis. Cool conditions specifically refer to operations at 4°C or less, or on ice. Furthermore, DNA polymerases that are designed to acquire DNA polymerase activity after being exposed once to high temperature conditions, are useful for preventing undesired reactions. An example of such a DNA polymerase is Hot Star Taq.

In the present invention, the step of synthesizing a complementary strand can be repeated many times as necessary. When a sufficient amount of the template nucleic acid exists, one cycle of complementary strand synthesis can produce elongation products in an amount sufficient for dissociation curve analysis. When there is a small amount of template nucleic acid, the same complementary strand synthesis can be repeated to provide the complementary strand in an amount required for analysis. In the present invention, when a complementary strand is synthesized by a complementary strand synthesis that utilizes each of the aforementioned primers, each of the primers is preferably designed such that it does not anneal to the previously synthesized complementary strand. Obtaining a mixture of elongation products comprising a variety of nucleotide sequences is a preferred condition of the present invention. However, if a different primer anneals to a pre-synthesized complementary strand, PCR-like complementary strand synthesis may be initiated. As a result, repeating the complementary strand synthesis will only accumulate large amounts of a double-stranded nucleic acid comprising a specific nucleotide sequence as the reaction product. In this invention, the presence of such reactive products may simplify the curves, and may decrease the analysis efficiency by disturbing the synthesis of the various products from the multiple regions.

When obtaining elongation products by repeating the same reaction conditions for the step of complementary strand synthesis, the conditions for each of the synthetic reactions are preferably kept uniform. By keeping the reaction conditions uniform, the size of the elongation products can be maintained within a constant range. This thus contributes to an increase in reproducibility of the identification methods of this invention.

As for the test nucleic acids, the nucleic acids synthesized in the above-mentioned step (1) may be DNAs, RNAs, or derivatives thereof. The most common method for sequence-dependent nucleic acid synthesis is complementary strand synthesis using primers and a DNA polymerase. In such cases, the nucleic acid to be synthesized is a DNA or derivative thereof. Oligonucleotides that can anneal to multiple regions in a test nucleic acid can be used as primers. The variations of the oligonucleotides that can be utilized as the primers for waveform production of this invention are described above.

Template-dependent transcription reactions that use RNA polymerase can be used for this invention. First, a DNA is synthesized in which the nucleotide sequence of a promoter, recognized by RNA polymerase, is added to the nucleotide sequence of multiple regions of the template nucleic acid. More specifically, complementary strand synthesis is performed by utilizing an oligonucleotide in which a promoter nucleotide sequence has been added to the 5' side of a primer comprising a nucleotide sequence complementary to the template. If the synthesized DNA is converted into a double strand, it can be utilized for a transcription reaction by RNA polymerase. When an RNA polymerase acts on a DNA comprising a promoter, a template-dependent transcription reaction proceeds under constant temperature conditions, and large amounts of RNA are transcribed. In this case, the synthesized nucleic acid is an RNA or derivative thereof.

In the present invention, the complementary strand synthesis products from the multiple regions of test nucleic acids synthesized in the aforementioned step (1), are specifically referred to as "elongation products". The present invention comprises the step of obtaining dissociation curves for a mixture of these elongation products.

Double-stranded nucleic acids formed by base pairing will eventually dissociate into single strands as temperature increases. At a specific temperature, dissociation into single-stranded nucleic acids is known to be rapid. The temperature at which double strands rapidly dissociate is called Melting Temperature (Tm). Tm is determined by the nucleotides of the nucleic acid, and by the components in the reaction solution. Therefore, in a reaction solution with the same composition, Tm is governed by the nucleotide sequence constituting the nucleic acid.

When a simple double-stranded nucleic acid exists in a highly pure state, similarly to a PCR reaction product, the Tm unambiguously determined. However, a complex of a variety of polynucleotides, which is similar to a mixture of elongation products, yields complex dissociation curves. The present invention was completed based on the finding that a template nucleic acid can be identified by comparing the dissociation curves caused by a mixture of elongation products.

Any method may be used to obtain dissociation curves. For example, the dissociation of a double-stranded nucleic acid into single strands can be detected using an intercalator. In the present invention, a compound that produces a signal on binding to a double-stranded nucleic acid is called an intercalator. Known intercalators are ethidium bromide, or the commercially available sybergreen (Molecular Probe).

Any of these compounds can be used for the dissociation curve analysis of this invention. For example, when sybergreen is added to a double-stranded DNA, the dissociation of the double-stranded structure can be detected by the decrease in fluorescence intensity.

Alternatively, a dissociation curve can be obtained by observing the state of dissociation of a double-stranded structure using electronic signals or optical methods such as absorbance measurements.

A dissociation curve can be obtained by changing, in a stepwise manner, the conditions for dissociating double-stranded nucleic acid structures composed of elongation products into single-stranded nucleic acids. The conditions used to dissociate double-stranded nucleic acid structures into single-stranded nucleic acids are discretionary. More specifically, dissociation into single-stranded nucleic acids can be accomplished by changing conditions such as temperature, hydrogen ion concentration, and denaturants. Of these conditions, changes in temperature are advantageous since temperature can be easily regulated, and the state of dissociation can be easily observed.

Analysis of dissociation curves of the elongation products yields dissociation curve waveform patterns. A "dissociation curve waveform pattern" refers to a dissociation curve that comprises information on multiple Tm's. Information on Tm's includes information on Tm values and Tm signal intensity. When a nucleic acid yields the same waveform pattern as the dissociation curve waveform pattern obtained for a certain nucleic acid, the two nucleic acids can be deemed identical. On the other hand, if the Tm combinations are different, or if the combinations of signal intensities for each Tm are different, the two nucleic acids are most likely to be different. In this way, in the identification methods of this invention, a dissociation curve waveform pattern yielded from a mixture of synthesis products from multiple regions of a test nucleic acid has a pattern that is unique to the test nucleic acid. In the present invention, such waveform patterns are called "genopatterns".

One type of double-stranded nucleic acid ordinarily has only a single Tm. For example, an amplification product obtained by a PCR method has a single Tm in most cases. Therefore, even if a portion of the nucleotide sequence differs between a test nucleic acid and a control nucleic acid to be compared, in order to detect that difference by comparing dissociation curves of PCR amplification products, a special configuration of reaction conditions and such would be required (JP-A 2002-325581). On the other hand, the present invention analyzes dissociation curves for mixtures of single-stranded nucleic acids, which are elongation products synthesized from multiple template nucleic acid regions.

The elongation product mixtures are complexes of polynucleotides that comprise various nucleotide sequences. In contrast to PCR products, such complexes do not comprise nucleotide strands that can pair over the full length in a completely complementary manner. Thus, these complexes are predicted to form unique higher order structures that depend on the nucleotide sequence, or to cause various mutual interferences due to partial pairing among the products. As a result, various complex dissociation curve waveform patterns can be obtained by dissociation and denaturation due to heating of the elongation products. Since various waveform patterns are obtained depending on the type of template nucleic acid, nucleic acids can be identified by analyzing waveform patterns.

Comparison and identification of the waveform patterns can be performed by visual comparative observation of patterns depicted as graphs. Furthermore, it is possible to objectively compare and quantitatively measure concordance rates using pattern recognition programs that use computer-readable electronic media on which waveform patterns are recorded. In addition to the concordance rate over entire waveform patterns of the ratio of changes in fluorescence intensity at each temperature, the following factors can be taken up as characteristics of the waveform patterns being compared: peak height in the pattern (ratio of fluorescence intensity change), peak position (melting temperature), peak number, peak width, order of the heights of multiple peaks, trough depth, position, number, width, order, and degree of slope, and such. Multiple factors can be selected, depending on the shape of the waveform pattern that is to be compared. Furthermore, the accuracy of recognition can be increased by weighing the comparative results for each characteristic according to their importance.

In the methods for identifying nucleic acids of this invention, a test nucleic acid is identified by comparing the dissociation curve waveform pattern obtained from the test nucleic acid with a reference dissociation curve waveform pattern. The reference waveform pattern can be obtained by performing the same operations as for the identification methods of this invention, using a standard nucleic acid sample as the sample.

In the present invention, a standard nucleic acid sample refers to a sample comprising a nucleic acid that is clearly the nucleic acid to be identified. For example, a nucleic acid whose entire nucleotide sequence has been elucidated can be used as a standard nucleic acid sample. Alternatively, even if the nucleotide sequence has not been determined, a nucleic acid obtained from a cell line of a microorganism can be included as a standard nucleic acid sample . Established microorganism cell lines can be regarded as cells that have been cloned. Viruses that have been established in the same way as microorganism cell lines, and a variety of cultured cell lines, are also useful as standard nucleic acid samples.

Nucleic acids for which the structures of regions for complementary strand synthesis are elucidated may also be used as standard nucleic acid samples. For example, even a large nucleic acid, such as the genome of an eukaryotic cell, can be utilized as a standard nucleic acid sample if the structures of the regions that are synthesized using the applied primers are known. In other words, by designing primers that allow synthesis of structurally known portions of a certain nucleic acid, the identification methods of this invention can be performed using this standard nucleic acid as a sample.

Reference waveform patterns can be produced when identifying test nucleic acids, or may be prepared in advance. It is convenient to prepare waveform patterns in advance, since only the test nucleic acids will need to be analyzed at the actual time of identification. The present invention provides methods for producing reference dissociation curve waveform patterns for identifying nucleic acids, wherein the methods comprise the steps of:
(1) synthesizing, as test nucleic acids, nucleic acids that comprise a nucleotide sequence complementary to multiple regions of a standard nucleic acid sample; and
(2) obtaining dissociation curves of a mixture of nucleic acids synthesized in step (1).

Producing reference dissociation curve waveform patterns for multiple types of standard nucleic acid samples, provides a means for identifying a greater variety of nucleic acids. These waveform patterns are preferably produced using a common primer complex. Reference waveform patterns produced under the same conditions using a common primer complex can be widely applied as reference waveform patterns for the waveform patterns of test nucleic acids analyzed under the same conditions.

By registering these reference waveform patterns on a computer-readable medium, they can be used as a reference dissociation curve waveform pattern database. In the present invention, "database" refers to a collection of information recorded on computer-readable media. "Computer-readable media" comprise magnetic disks, magneto-optical disks, optical disks, or memory circuits. Furthermore, data recording devices connected through a network, such as the Internet, are also included as computer-readable media.

Using a database of the present invention, users can obtain a reference waveform pattern through the network to perform the methods for identifying nucleic acids of this invention. Alternatively, users can send a produced dissociation curve waveform pattern for a test nucleic acid to a server, the pattern can be verified against the database, and a result can be provided to users from the server.

Furthermore, the present invention relates to methods for producing primer complexes for waveform production. The present inventors succeeded in designing various types of primer complexes for waveform production, which may be used in the methods for identifying nucleic acids of this invention. The primer complexes for waveform production are mixtures of specific primers and ambiguous primers. Several ambiguous primers, designed by the present inventors, are useful as novel primer complexes for waveform production, and enable simultaneous complementary strand synthesis of multiple regions of template nucleic acids. Of these ambiguous primers, those ambiguous primers structured such that ambiguity changes in a stepwise manner towards the 5' side can be referred to as structurally novel primers. A primer complex for waveform production which comprises such ambiguous primers can be obtained by a method for producing a primer complex for waveform production,
wherein the primer complex comprises:
a specific region, which comprises the 3' end of the oligonucleotide and consists of a nucleotide sequence complementary to the target region; and
an ambiguous region, which is positioned to the 5'-side of the specific region, and comprises a nucleotide sequence wherein a nucleotide comprised in the nucleotide sequence complementary to the target region is substituted with a nucleotide other than the nucleotide; and
wherein the method comprises the steps of:
a) synthesizing the specific region; and
b) synthesizing the ambiguous region by binding a nucleotide of a nucleotide sequence complementary to the target region, to a mixture of random nucleotides other than the nucleotide, selected from adenine, cytosine, guanine, and thymine.

The ambiguous primers of this invention can be synthesized by utilizing the same synthesis reactions as for degenerate primers. For example, methods for nucleic acid synthesis including the phosphotriester method, phophoramidite method, or phosphonate ester method have been reported. Of these, the phosphoramidite method is widely used as a basis to enable automated synthesis by a DNA synthesizer. For synthesis of DNAs using a DNA synthesizer, solid phase synthesis techniques are generally used. Specifically, DNAs are synthesized by chemically bonding mononucleosides of the nucleotide sequence of interest in the 3' to 5' direction, one base at a time, to nucleosides bonded to a resin at the 3' position. Herein, if a mixture of multiple nucleotides is provided as the mononucleosides, different nucleotides will bond in that position. In the chemical synthesis of DNA, when the concentration of each of the nucleotides is the same, bonding reactions are presumed to occur with equal probability. Therefore, when a mixture comprising equal concentrations of adenine and guanine is provided, for example, DNAs comprising two types of nucleotide sequences, in which adenine or guanine exists at a certain position, will be synthesized. More specifically, the use of a mixture of two types of nucleotides allows a 2 ambiguous region of this invention to be synthesized. Similarly, an N region and a 3 ambiguous region are synthesized by a mixture of four types and three types of nucleotide, respectively.

In the present invention, ambiguous primers are synthesized, based on the nucleotide sequence of the specific primer, by successively reacting a mixture of the ambiguity code nucleotides at each position. In this way, specific primers and ambiguous primers can be synthesized simultaneously, as a complex. Synthesized complexes can be purified by HPLC as necessary, and used for complementary strand synthesis.

Each region of an ambiguous primer can be successively synthesized, or the whole ambiguous primer can be obtained by linking regions that are synthesized separately. For example, when the ambiguous primers of this invention are constituted by a specific region (comprising the 3' end) and an ambiguous region (comprising the 5' end), the two can be separately synthesized and then linked to yield the ambiguous primer. Methods for linking oligonucleotides using enzymes such as T4 ligase are well known.

Furthermore, when the ambiguous region is made up of multiple regions with different ambiguities, each of the regions may be separately synthesized. Separately synthesized regions with different ambiguities can be constructed into an ambiguous primer that comprises a subject nucleotide sequence, by linking them according to the design of the ambiguous primer. For example, the N regions are identical regardless of the nucleotide sequence of the specific primer. Therefore, for any template nucleotide sequence, the required length of the N region can be synthesized in advance. Furthermore, since the number of nucleotide sequence combinations is limited for the 3 ambiguous region, synthesizing these oligonucleotides in advance is meaningful.

By combining each of the components necessary for the nucleic acid identification methods of this invention, a kit for nucleic acid identification can be produced. More specifically, the present invention relates to kits for identifying a nucleic acid, wherein the kit comprises the components of:
(1) a primer complex for waveform production, that comprises a mixture of one or more types of primers comprising nucleotide sequences complementary to multiple regions of a test nucleic acid;
(2) a DNA polymerase that catalyzes template-specific synthesis of a complementary strand; and
(3) a substrate for complementary strand synthesis.

Primer complexes such as those described above may be used in the kits of this invention. Similarly, the kits of this invention can comprise the DNA polymerases and substrates for complementary strand synthesis that can be used in this invention. The kits of this invention may additionally comprise the dissociation curve waveform pattern of a positive control and/or nucleic acids to be identified. Furthermore, the kits of this invention may include intercalators that can be used to analyze the dissociation curves. Examples of intercalators include sybergreen.

Each of the components of a kit of this invention can be provided by pre-loading them into a reaction vessel. More specifically, the primer complex for waveform production, DNA polymerase, nucleotide substrate, buffers of the reaction solution, intercalators, and such, for complementary strand synthesis can be loaded into a reaction vessel in advance. The amount of each component can be set according to the final concentration of reaction solution. Each component may be in a dry or liquid state. Generally, proteins such as enzymes are said to more readily maintain their activity during storage when converted into a dry form. The methods of this invention can be simply performed by the steps of adding a sample solution comprising a nucleic acid into such a reaction vessel; and then incubating it under the required temperature conditions. Containers with many independent reaction spaces can be utilized as the reaction vessel used in the kits of this invention. For example, a reaction vessel such as a 96 (12 x 8)-well microtiter plate is useful for the kits of this invention.

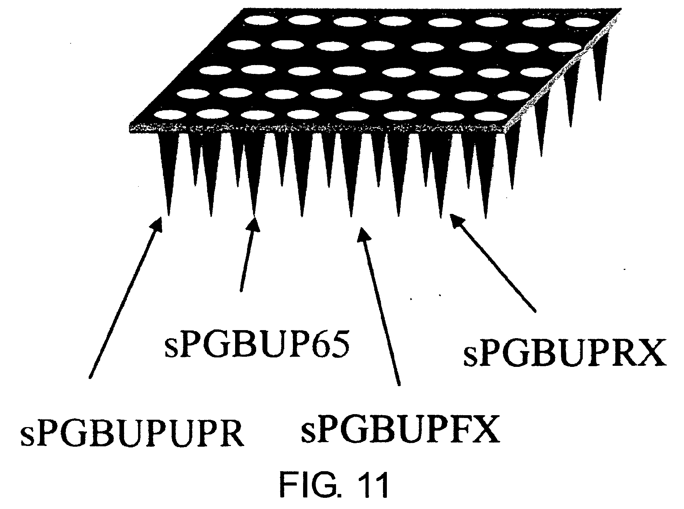

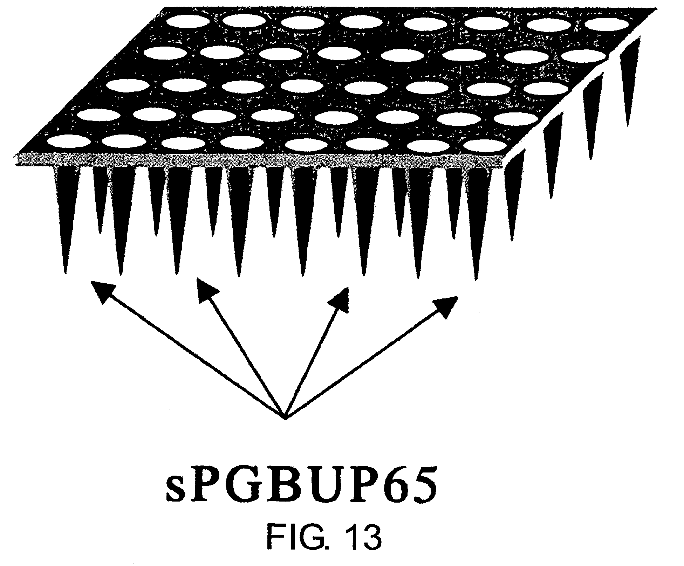

For example, the same primer complex for waveform production can be loaded into the multiple reaction spaces to obtain a kit for simultaneously identifying multiple nucleic acid samples (Fig. 13). Alternatively, each of the multiple types of primer complexes for waveform production can be loaded into separate reaction spaces to obtain kits for producing multiple waveform patterns for the same nucleic acid sample (Fig. 11). More specifically, a kit can be used to identify twelve samples when using eight types of primer complex for waveform production in a reaction vessel comprising 96 wells, such as the aforementioned microtiter plate.

The methods for identifying nucleic acids of this invention can be applied to any nucleic acid requiring identification. The following describes specific examples of nucleic acids for which various advantages can be expected on applying the methods for identifying nucleic acids of this invention.

The present invention is useful for differentiating groups of microorganisms having a high genetic homogeneity. The methods for identifying nucleic acids of this invention enable slight differences in multiple regions of nucleic acids to be comprehensively differentiated. For example, by analyzing the dissociation curves of elongation products obtained for multiple regions, mutually unique waveform patterns can be obtained even from a group of microorganisms that comprise nucleotide sequences that are highly homologous over the entire genome. As a result, waveform patterns that are unique to each bacterial strain can be easily distinguished.

For example, acid-fast bacteria species such as *Mycobacterium tuberculosis* are representative groups of microorganisms that require differentiation. In order to identify acid-fast bacteria using PCR, primers for many regions must be designed, and the amplification reaction must be repeated. On the other hand, by utilizing the present invention, all of the information necessary for identification can be obtained from a single reaction vessel, by using a primer complex that can yield elongation products from multiple regions. Alternatively, by using two reaction vessels to apply different primer complexes to one test sample, the reliability of the identification result can be increased.

Similar advantages can be expected when testing for diseases caused by genetic abnormalities, such as cancer. Abnormality of a specific gene such as p53 has been pointed out as a possible cause of cancer. However, the genetic abnormalities which cause cancer have not been entirely elucidated. In theory, abnormalities in an unknown gene cannot be discovered using PCR. However, by utilizing this invention, the conditions for producing a waveform pattern unique to cancer cells can be easily found, even if the causative gene has not been identified.

Furthermore, the present invention can be utilized in polymorphism analysis. Single nucleotide polymorphisms, such as SNPs, and polymorphisms of repeated sequences, such as satellite markers, are known examples of polymorphisms. The relationships between these polymorphisms and various genotypes have been elucidated one after the other, and a lot of information has been accumulated. Various methods for detecting polymorphisms are known. However, whilst many of these methods can detect a specific polymorphism, they usually cannot detect multiple polymorphisms simultaneously. For example, a DNA array that has accumulated a high density of various DNA probes is an effective tool for simultaneously analyzing multiple polymorphisms. However, even when using a DNA array, information on nucleotide sequences that are not comprised as probes will not be obtained as long as there is a dependence on probe hybridization. Furthermore, at least for now, DNA arrays are expensive analysis devices. While DNA arrays are widely used as research tools, a more inexpensive and practical analysis tool is required for routine use in nucleic acid identification methods.

In contrast, if regions comprising polymorphisms are produced as elongation products by the methods of this invention, the many polymorphisms comprised in the elongation products may be comprehensively compared. This means that genetic diagnosis techniques based on polymorphisms may be performed far more efficiently than for known methods. Furthermore, even if the polymorphism itself is unknown, the methods for identifying nucleic acids of this invention can distinguish the nucleic acid by changes in the waveform pattern. Furthermore, the methods of this invention can be performed inexpensively by utilizing reagents that are generally used for methods of nucleic acid synthesis.

Generally, the phrase "identification of nucleic acids" refers to confirming whether a test nucleic acid is identical to another nucleic acid. Different types of cells typically comprise different nucleic acids, so the present invention can thus differentiate cell types. Furthermore, even if the cells are the same type, the present invention can be applied to confirm the identity of a cell' s condition when the nucleic acids are in a different state due to that condition. For example, nucleic acids are known to degrade due to cell death. Cell viability can be tested based on the present invention by using this phenomenon. More specifically, cell death can be confirmed by confirming the disappearance of a waveform pattern obtained from a live cell. For example, in PCR and the like, as long as an amplification target region exists, identification results of the cells obtained, regardless of the viability. However, with this invention, the nucleic acid condition can be captured in a more multifaceted manner, and thus cell death can be seen as a change in the waveform.

Fig. 11 shows an example of the composition of a kit for identifying nucleic acids of this invention. The kit of Fig. 11 refers to a kit of this invention that utilizes a reaction vessel equipped with multiple wells. In this example, four types of primer complexes for waveform production, sPGBUP65, sPGBUPUPR, sPGBUPFX, and sPGBUPRX are individually pre-loaded into separate wells. Furthermore, the other reagent components necessary for complementary strand synthesis by DNA polymerase are also pre-loaded into each well. Therefore, the identification method of this invention can be performed by simply adding a test nucleic acid and adjusting predetermined conditions. The four types of primer complexes for waveform production shown in this example, sPGBUP65, sPGBUPUPR, sPGBUPFX, and sPGBUPRX, are those individually designed for different regions. More specifically, by using multiple regions of a test nucleic acid as target regions, as shown in Fig. 12, four types of primer complexes for waveform production can be designed. Such combinations result in more types of waveform pattern. More specifically, the types of nucleic acids that can be identified by the present invention can be increased.

In addition, the present invention provides methods for comprehensively differentiating differences in the nucleotide sequences of each gene of a target gene cluster comprising a conserved nucleotide sequence. Using PCR, it is difficult to design a primer individually specific to each gene that comprises a highly conserved nucleotide sequences. Therefore, it is difficult to analyze such gene clusters using PCR. On the other hand, performing complementary strand synthesis for multiple regions is a condition of this invention, the synthesis of multiple regions using a small number of primers is instead considered to be a preferable condition. More specifically, gene clusters comprising a conserved nucleotide sequence are preferable as targets for the identification methods of this invention. In this invention, the phrase "gene cluster comprising a conserved nucleotide sequence" refers to a condition in which multiple genes comprise homologous nucleotide sequences. Such gene clusters are also called gene families.

For example, the present invention can be applied to the identification of the gene family of drug metabolizing enzyme, cytochrome P450. The cytochrome P450 gene family is a gene cluster comprising many genes highly homologous in structure. In addition, very many polymorphisms exist in this gene cluster. To extensively identify each polymorph using PCR, in principle, complementary strand synthesis must be carried out as many times as the number of types of polymorphs to be analyzed. That is, methods using PCR to identify the cytochrome P450 gene family are inefficient. Furthermore, since the individual genes are highly homologous in structure, it would be difficult to design specific primers for PCR. On the other hand, in the methods of this invention, many polymorphic regions can be identified in one reaction by synthesizing the regions that comprise the polymorphic regions of each gene family as elongation products.

Fig. 13 shows a kit of this invention in which the same type of primer complex for waveform production (sPGBUP65) is loaded into multiple wells. Kits with such a constitution are useful for simultaneously identifying multiple test nucleic acids.

The primers for waveform production and the methods for identifying nucleic acids using the primers of this invention include the following embodiments:
(1) Primers for waveform production, wherein a primer comprises i) a nucleotide strand at the 3' end, that is complementary to any specific or non-specific region of a nucleic acid, and comprises single nucleotides positioned in each nucleotide strand, and ii) a nucleotide strand at the 5' end, which may be complementary to said region and has multiple nucleotides positioned in each nucleotide strand, and is characterized by a nucleotide strand that increases in complementarity in a stepwise manner in the 5' to 3' direction, and a sequence in which stability decreases in a stepwise manner.
(2) The primers for waveform production of (1), wherein the nucleotide strand of a primer comprises a first region composed of a code indicating any four nucleotides including adenine, cytosine, guanine, and thymine; a second region comprising a code indicating any three of the nucleotides; and a third region comprising a code indicating any two of the nucleotides; where each of the nucleotides of the first, second, and third regions can be designed to be any length.
(3) The primers for waveform production of (1), wherein a primer comprises ten to 30 nucleotides, and the proportion of nucleotides complementary to a full length primer is 0.12 to 0.88.
(4) A method for amplifying a nucleic acid that amplifies a partial nucleotide sequence of a nucleic acid, the method comprising the steps of:
   selecting any specific or non-specific region in the above mentioned nucleic acid;
   annealing a primer for waveform production of (1) to multiple regions comprising at least the region in the above mentioned nucleic acid, wherein the primer comprises a nucleotide strand that may have complementarity to the nucleotide strand complementary to the above-mentioned region; and
   performing nucleotide strand synthesis in the presence of the primers and polymerase, using the multiple regions as templates.
(5) The method for amplifying a nucleic acid of (4), wherein the annealing temperature is set at 49°C or lower in the step of annealing the primer for waveform production to any specific or non-specific region in the nucleic acid.
(6) The method for amplifying a nucleic acid of (4), wherein in the step of performing nucleic acid amplification, using the primers for waveform production, of any specific or non-specific region in a nucleic acid, only one type of sense primer or antisense primer is used, and only one strand, either the sense strand or the antisense strand of the nucleic acid, is amplified, and the nucleic acid amplified at this time is not reused as a template in the subsequent amplification step.
(7) A method for identifying a nucleic acid by amplifying a partial nucleotide sequence of a nucleic acid to identify nucleic acids, wherein the method comprises the steps of:
   selecting any specific or non-specific region in the nucleic acid;
   annealing a primer for waveform production of (1) to the multiple regions comprising at least the region in the above-mentioned nucleic acid, wherein the primer comprises a nucleotide strand that may have complementarity to the nucleotide strand complementary to the above-mentioned region;
   performing a nucleotide strand synthesis using the above-mentioned multiple regions as templates in the presence of the primers and polymerase;
   promoting the obtained multiple nucleic acid amplification products to form various mutual interferences such as higher order structures and contaminants; and
   heating the mutual interference-producing nucleic acid amplification products, to identify nucleic acids based on the waveform pattern of the dissociation curve obtained from the dissociation and denaturation of the nucleic acid amplification products.

In addition, the primers for waveform production, and methods for identifying a nucleic acid using these primers of this invention include the following embodiments:
(1) Methods for amplifying nucleic acids and methods for identifying nucleic acids, wherein different regions of a double-stranded nucleic acid of interest are individually amplified at the same time, each using different primers in multiple independent liquid reaction systems; and the character of a double-stranded nucleic acid of interest is identified from a combination of melting temperatures or dissociation patterns obtained when dissociating or denaturing the amplification products into single-stranded nucleic acids by heating.
(2) Methods for amplifying nucleic acids and methods for identifying nucleic acids, wherein equivalent regions of a number of different double-stranded nucleic acids of interest are individually amplified at the same time, each using the same primers in multiple independent liquid reaction systems, and the characters of a number of different double-stranded nucleic acids of interest are identified at the same time from melting temperatures or dissociation patterns obtained when dissociating or denaturing the amplification products into single-stranded nucleic acids by heating.
(3) Methods for amplifying nucleic acids and methods for identifying nucleic acids, wherein the characters of a number of different double-stranded nucleic acids of interest are identified at the same time from melting temperatures or dissociation patterns obtained when dissociating or denaturing the amplification products obtained from nucleic acid amplifications performed separately in a number of isolated liquid reaction systems.
(4) The methods for amplifying nucleic acids and methods for identifying nucleic acids of any one of (1) to (3) , wherein the liquid reaction system is a liquid-phase DNA chip.
(5) Methods for amplifying nucleic acids and methods for identifying nucleic acids, such that the character of the double-stranded nucleic acid of interest in (1), (2), or (3) is identified using one or more specific or non-specific primers against the target nucleic acid.
(6) Methods for amplifying nucleic acids and methods for identifying nucleic acids, such that the character of the double-stranded nucleic acid of interest in (1), (2), or (3) is identified by performing nucleic acid amplification using DNA polymerase (DNA synthase) containing Taq DNA polymerase (thermostable DNA synthase).
(7) Methods for amplifying nucleic acids and methods for identifying nucleic acids, such that the character of the double-stranded nucleic acid of interest in (1), (2), or (3) is identified by amplifying a single strand of either the sense strand or the antisense strand of the nucleic acid, and by using a method for amplifying a nucleic acid that does not reuse the amplified nucleic acid as a template for subsequent amplification cycles.
(8) Methods for amplifying nucleic acids and methods for identifying nucleic acids, such that the character of the double-stranded nucleic acid of interest in (1), (2), or (3) is identified in the step of nucleic acid amplification, particularly in the step of annealing the primer by performing the reaction at an annealing temperature of not more than 49°C.
(9) Methods for amplifying nucleic acids and methods for identifying nucleic acids, such that the character of the double-stranded nucleic acid of interest of (1), (2), or (3) is identified using an intercalator for the detection of melting temperatures or dissociation patterns obtained during dissociation or denaturation into single-stranded nucleic acids.

### Brief Description of the Drawings

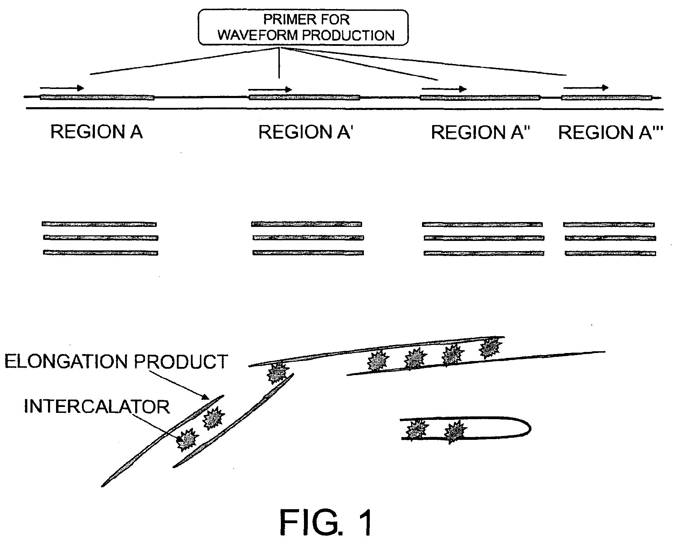

Fig. 1 schematically describes a method for synthesizing a complementary strand of this invention. Fig. (A) shows that multiple regions of the test nucleic acid are synthesized by this invention. Fig. (B) shows that multiple elongation products form various mutually interfering structures.
Fig. 2 shows the structure of an ambiguous primer used in this invention.

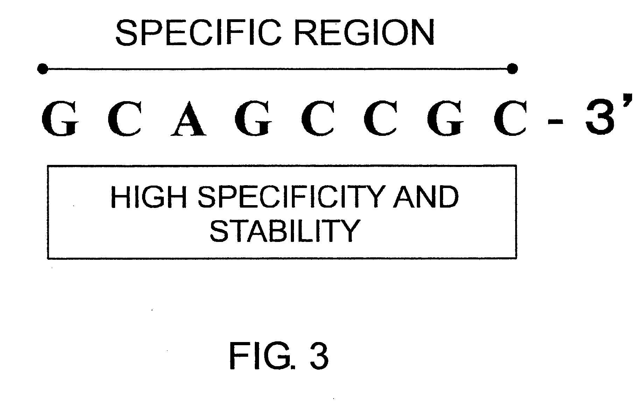

Fig. 3 shows an example of the sequence of the specific region of an ambiguous primer.
Fig. 4 shows an example of the sequence of the ambiguous region of an ambiguous primer.
Fig. 5 schematically shows the complementary strand synthesis of this invention by BAUP65, a primer complex for waveform production.
Fig. 6 shows dissociation curve waveform patterns. Fig. (A) shows the waveform pattern of elongation products produced by a complementary strand synthesis of this invention, and Fig. (B) shows the waveform pattern of products amplified by PCR. In the figures, the x-axis indicates the fluorescence intensity differential, and the y-axis indicates temperature (C°).
Fig. 7 is a set of photographs showing the electrophoretic analysis results of the reaction products. Fig. (A) shows the electrophoretic analysis results of elongation products obtained using an ambiguous primer complex of this invention, and Fig. (B) shows the electrophoretic analysis results of amplification products produced by PCR amplification. M indicates the molecular weight marker. Lanes (1) to (3) each indicate the result from reaction products that were obtained using the template DNAs from *Camphylobacter jejuni, Haemophilus influenzae*, and *Salmonella typhimurium*, respectively.
Fig. 8 shows changes in the dissociation curve waveform pattern due to differences in annealing temperature. Figs. (A) , (B) , and (C) show the waveform patterns obtained when annealing using *E*. *coli* at 55°C, 40°C, and 25°C respectively.
Fig. 9 shows changes in the dissociation curve waveform pattern due to differences in annealing temperature. Figs. (A) , (B) , and (C) show the waveform patterns obtained when annealing using *S*. *aureus* at 55°C, 40°C, and 25°C respectively.
Fig. 10 shows changes in the dissociation curve waveform pattern depending on the presence or absence of an ambiguous region. Fig. (A) shows the waveform pattern in the presence of an ambiguous region, and Fig. (B) shows the waveform pattern in the absence of an ambiguous region.
Fig. 11 shows a sample composition of a kit for nucleic acid identification of this invention. In the figure, sPGBUP65, sPGBUPUPR, sPGBUPFX, and sPGBUPRX are the names of primer complexes for waveform production.
Fig. 12 schematically shows the positional relationship of "target regions" in a test nucleic acid, where the "target regions" were set in order to design the four primer complexes for waveform production: sPGBUP65, sPGBUPUPR, sPGBUPFX, and sPGBUPRX.
Fig. 13 shows a sample composition of a kit for multiple and simultaneous nucleic acid identification of this invention.
Fig. 14 shows the differences in waveform patterns obtained from three types of primers for waveform production when using DNAs extracted from *E. coli, S. aureus,* and *B. cereus* as the test nucleic acids. In the figure, the vertical axis indicates the fluorescence intensity differential, and the horizontal axis indicates the temperature (C°). sPGBUP65, sPGBUPUPR, and sPGBUPFX are the names of primers for waveform production.
Fig. 15 shows the influence the addition of a stabilizer during complementary strand synthesis has on waveform pattern. Fig. 15(A) shows the waveform pattern in the absence of a stabilizer, and Fig. 15(B) shows the waveform pattern when a stabilizer is added. In the figures, the x-axis indicates the fluorescence intensity differential, and the y-axis indicates temperature (C°).
Fig. 16 shows the influence the influence the addition of a stabilizer during complementary strand synthesis has on complementary strand synthesis. Figs. 16(A) and 16(B) are photographs showing the electrophoretic analysis results of the synthesis products in the absence and presence of a stabilizer, respectively. Figs. (A) and (B) show a 200 bp ladder in the left lane, and the electrophoretic analysis results of the samples (duplicates) in the two right lanes.

### Best Mode for Carrying Out the Invention

Hereinafter, the present invention is specifically illustrated with reference to Examples.

### [Example 1] Design of ambiguous primers

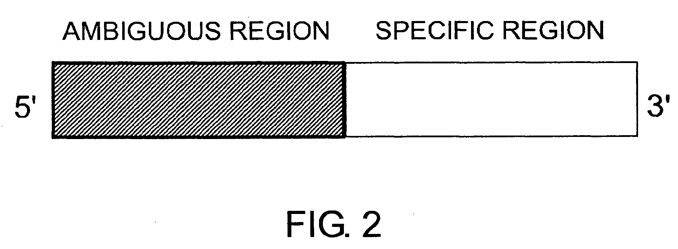

As mentioned above, the present invention provides ambiguous primers comprising a specific region and an ambiguous region. This concept is shown in Fig. 2. The ambiguous primers represented by Fig. 2 can anneal not only to a specific nucleotide sequence but also to analogous sequences that are similar to this sequence since they have an ambiguous region at their 5' end. When actually using a primer complex for waveform production that has such a structure, preferably, the conditions indicated in (a) to (e) below are adjusted:
(a) primer length
(b) sequence of the specific region
(c) sequence of the ambiguous region
(d) primer amounts
(e) annealing temperature

Accordingly, items (a) through (e), mentioned above as examples of structural factors of the ambiguous primers of this primer complex for waveform production, and reaction conditions, were examined in order.

### (a) Adjustment of primer length

First, in order to examine the effect of a full-length primer on synthesis efficiency, complementary strand synthesis was carried out by performing 50 cycles of a protocol involving 98°C for two seconds, 40°C for 20 seconds, and 72°C for 20 seconds, using primers with a 1:1 ratio of specific region to ambiguous region. The waveforms of the dissociation curves for the elongation products obtained by complementary strand synthesis were analyzed, and the effects of ambiguous primer structure on the results were compared. The ambiguous primers were evaluated based on two indices: specificity and stability.

Herein, "high specificity" means that annealing to regions other than those selected as target regions is difficult. In the present invention, high specificity of the ambiguous primers reduces the diversity of elongation products. As a result, waveform diversity is lost. More specifically, this means that preferable ambiguous primers in this invention are ambiguous primers whose specificity is reduced to some extent.

On the other hand, stability refers to the reproducibility of annealing by each primer constituting an ambiguous primer. As previously mentioned, ambiguous primers are a complex of oligonucleotides comprising different nucleotide sequences. Individual oligonucleotides in the complex should anneal to nucleotide sequences complementary to their nucleotide sequences. However, when the ambiguous region is too long, the resulting amount of oligonucleotide corresponding to the nucleotide sequence of interest is reduced. Therefore, there is an increased possibility that sufficient complementary strand synthesis will not take place.

As a result of this examination, preferable primer specificity or stability was observed when the full-length primer was 16- to 24-mer. These conditions maximized the diversity of the waveform of the elongation products.

On the other hand, although primer specificity or stability increased when the full-length primer was 24 to 30 mer, the diversity of the resulting dissociation curve waveform decreased. An increase in specificity was considered to result in insufficient progress of complementary strand synthesis of regions other than those selected as target regions. When the full-length primer was 10 to 16 mer, the diversity of the waveform increased, while specificity and stability decreased. When the full-length primer was 9 mer or shorter, synthesis did not occur. The conclusion from these results is that in this invention, ambiguous primers with a length in the range of 10 to 30 mer can be used, and of these, 16 to 24 mer are preferred.

Next, the proportion of specific region and ambiguous region over the full length of the primer was examined. As a result, when the proportion of ambiguous region over the full length of the primer was 0.33 to 0.55, the stability and diversity of the elongation products was maximized. In terms of the number of nucleotides, preferable results are yielded by an ambiguous region which is 8 to 13 mer in a full-length ambiguous primer of 24 mer.

On the other hand, when the proportion of the ambiguous region over the full length of the primer was 0.55 to 0.88, waveform diversity increased, but stability decreased. In terms of numbers of nucleotides, this condition refers to an ambiguous region which is 14 to 25 mer, and a specific region of 4 to 7 mer. Waveform diversity was lost when the proportion of the ambiguous region over the full length of the primer was 0.12 to 0.33. In terms of numbers of nucleotides, this condition refers to an ambiguous region which is 4 to 7 mer, and a specific region which is 14 to 25 mer.

Furthermore, when the proportion of the ambiguous region over the full length of the primer was 0.89 or more, so-called "random" primers were produced, and normal synthesis did not take place. In terms of numbers of nucleotides, this condition refers to, for example, an ambiguous region which is 25 mer or longer, and a specific region which is 3 mer or shorter. Furthermore, when the proportion of the ambiguous region occupying the entire primer is 0.11 or less, synthesis reactions were observed to be the same as those using ordinary PCR primers. More specifically, approximately only one kind of complementary strand synthesis product was observed. This condition, in terms of numbers of nucleotides, refers to an ambiguous region which is 3 mer or shorter, and a specific region which is 25 mer or longer, for example.

The following explanation will be made assuming the proportion of the ambiguous region over the full length of the primer to be 0.33 to 0.55.

### (b) Sequence of the specific region

The specific region comprises a nucleotide sequence complementary to a target region selected from a test nucleic acid. The length of the specific region is preferably 8 to 13 mer when the full length of the ambiguous primer is 16 to 24 mer. Furthermore, the GC%, which is the proportion of guanine (G) or cytosine (C) in the 3'-end five nucleotides, is preferably 50% or more. Furthermore, the stability of the 3' end should be -5.5 to -9.5 kcal/mol or more. Annealing is possible even when a mismatch of 50% or less exists in nucleotides other than the three nucleotides at the 3' end. In this case, if the stability of the mismatch portion is -12.0 kcal/mol or less, the decrease in the efficiency of synthesis may be ignored.

### (c) Sequence of the ambiguous region

The ambiguous region is a region positioned to increase the number of primers that can anneal to regions other than those target regions selected from a test nucleic acid. The ambiguous region comprises a nucleotide sequence in which nucleotides complementary to the aforementioned target region are substituted with other nucleotides. The types of nucleotides used for the substitution may be any of one to three types of nucleotides. In the present invention, the ambiguity codes shown in Table 1 are used to denote the nucleotide substitutions. The structure of an ambiguous primer can be indicated by a nucleotide sequence such as that shown in Fig. 4. An ambiguous primer comprising the nucleotide sequence shown in Fig. 4 comprises a structure with decreasing ambiguity of the ambiguous region in a stepwise manner in a 5' to 3' direction. The length of the ambiguous region is preferably 8 to 13 mer when the full-length primer is 16 to 24 mer.

**Table 1.**

| Ambiguity Codes | |
|---|---|
| Code | Nucleotides |
| M | Adenine (A) or Cytosine (C) |
| R | A or Guanine (G) |
| W | A or Thymine (T) |
| S | C or G |
| Y | C or T |
| K | G or T |
| V | A, C or G |
| H | A, C, or T |
| D | A, G, or T |
| B | C, G, or T |
| N | A, C, G, or T |

### (d) Primer amounts

A complex of ambiguous primers comprising the above-mentioned ambiguous regions is a complex of oligonucleotides comprising nucleotide sequences that are practically different. Therefore, if a "2 ambiguity code" is comprised, in practice, this means that two types of oligonucleotides comprising different nucleotide sequences are included. Therefore, the effective rate of annealing to a template nucleic acid becomes 1/2.

For example, the nucleotide sequence, SATT, comprising a "2 ambiguity code", S (indicating C or G), practically refers to oligonucleotides comprising two types of nucleotide sequences, CATT and GATT. In this case, the effective amount of each primer becomes 1/2. Similarly, when using a "3 ambiguity code" or a "4 ambiguity code", the effective amount of primer becomes 1/3 or 1/4, respectively. More specifically, the sequence, BATT, comprising a "3 ambiguity code", B (indicating C, G, or T) , refers to oligonucleotides comprising three types of nucleotide sequences: CATT, GATT, or TATT.

Accordingly, in practice, the effective amount of primers must be considered when calculating primer amounts. More specifically, the inverse of the effective amount of primer is multiplied by the minimum amount of primer, and this is divided by the number of nucleotides in the ambiguous region and the mismatch ratio, four. In Fig. 4, since there are two "4 ambiguity codes", four "3 ambiguity codes", and two "2 ambiguity codes", the effective amount of primer is 1/5184 (= (1/4)² x (1/3)⁴ x (1/2)²). Furthermore, the number of nucleotides in the ambiguous region is 8. Therefore, when the minimum amount of primer is 1 (pmol/50 µL), the amount of primer to be added in practice is approximately (1 x 5184 ÷ 8 ÷ 4) = 162 (pmol/50 µL). This method for calculating the amount of primer is only an example, and such methods are not limited thereto.

### (e) Annealing temperature

To examine the annealing temperature, first, the Tm value of a primer was calculated. Tm value refers to a temperature at which 50% of the nucleic acids comprising nucleotide sequences complementary to each other are base paired. More specifically, the Tm value of a specific region was calculated according to the "2 (A+T) +4 (G+C) " method; and that of an ambiguous region was calculated from the averaged "2(A+T)+4(G+C)" method. The Tm value of the full-length primer was then calculated by integrating these values.

For ordinary PCR, annealing temperatures are approximately 5°C lower than Tm values. In this invention, the annealing temperatures are preferably 20°C lower than Tm values, to diversify waveform patterns. Low temperatures facilitate the annealing of short primers. Experimentally, the diversity of the dissociation curve waveform increased remarkably at or below 40°C. On the other hand, only random priming occurred at or below 15°C, and synthesis hardly took place.

### [Example 2] Identification of bacterial genes

### (2-1) Synthesis and identification of bacterial genes

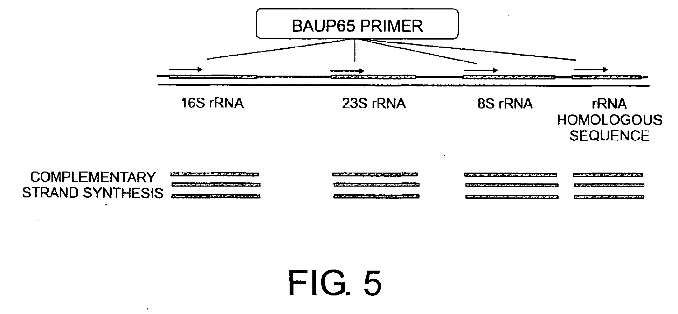

BAUP65 primer (nnvhdbssga tccaaccgc/ SEQ ID NO: 6) is a primer complex for waveform production that only synthesizes bacterial genes, and emphasizes the differences in dissociation curve waveform patterns between bacterial strains. BAUP65 primer was prepared for use in complementary strand synthesis and nucleic acid identification. More specifically, the primer was designed by the steps of: selecting, as the sequence of the specific region, eleven nucleotides from a DNA sequence encoding bacterial 16s ribosomal RNA (rRNA), where the nucleotides are conserved in approximately 3,000 bacterial strains; and then ligating an eight-nucleotide ambiguous region to this. As in the schematic representation of Fig. 5, in addition to the sequence encoding 16s rRNA, sequences homologous to 16s rRNA, such as 23s rRNA and 8s rRNA, are also expected to be simultaneously synthesized by complementary strand synthesis.

*Campylobacter jejuni, Haemophilus Influenzae,* and *Salmonella typhimurium* were selected as the bacteria to be used, and DNAs were extracted by using ISOGEN-LS (Nippon Gene), according to the instructions. Complementary strand synthesis using each of these sample DNAs as templates was performed by preparing a reaction solution with the composition shown below in Table 2, and using 25 µL of this solution.

**Table 2.**

| Composition of complementary strand synthesis solution | |
|---|---|
| Formulation of the reaction solution | Formulated amount |
| Sample DNA solution (comprising 1 µg of DNA) | x µL |
| Sterilized distilled water (comprising a stabilizer) | y µL |
| 10x PCR buffer | 5 µL |
| 5x SyGreen (sybergreen) buffer | 10 µL |
| dNTP (10 mM) | 4 µL |
| 25 mM MgCl₂ | 3 µL |
| BAUP65 primers (10 pmol/µL) | 18 µL |
| Taq DNA polymerase (5 U/µL) | 0.5 µL |
| Total | 50 µL |

In Table 2, the amount of sample DNA solution used contained 1 µg of DNA, such that x µL + y µL = 9.5 µL. This was prepared by adding sterilized distilled water (containing a stabilizer).

Waveforms of the dissociation curves of complementary strand synthesis products were observed by the steps of: synthesizing the complementary strands by a protocol with conditions of "70 cycles of 98°C for two seconds, 25°C for 40 seconds, and 72°C for 10 seconds"; and monitoring the products using Smart Cycler (TaKaRa) for one second at each temperature between 70°C to 94°C, increasing the temperature by 0.1°C.

As a comparative example, instead of using BAUP65 primers (SEQ ID NO: 6) , sample DNA was amplified by an ordinary PCR method, using a sense primer (cagcagccgc ggtaatac/ SEQ ID NO: 7) and an antisense primer (acgacacgag ctgacgac/ SEQ ID NO: 8) to amplify the sequence encoding a portion of 16s rRNA.

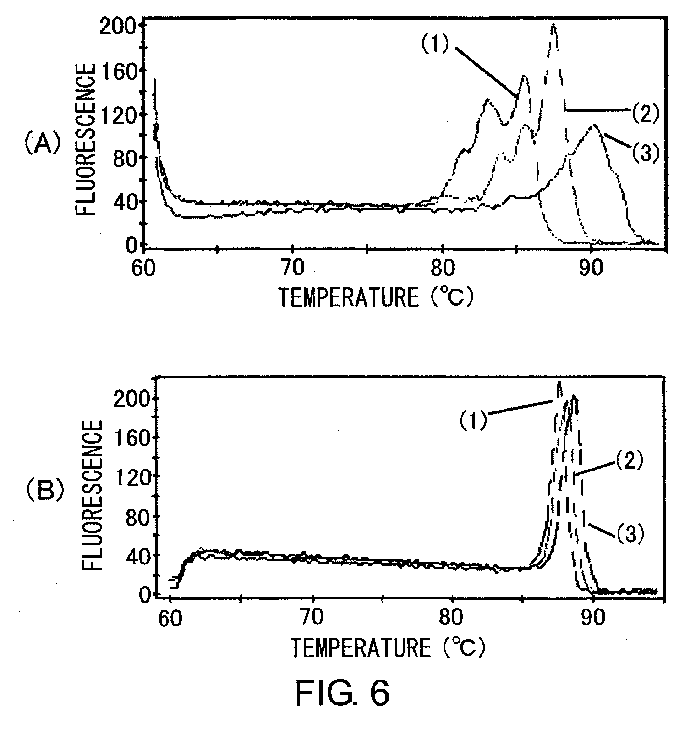

Figs. 6(A) and (B) show the dissociation curve waveform patterns obtained from this invention and from the PCR method, respectively. In Fig. 6, (1) is derived from *C. jejuni;* (2) is derived from *H. Influenzae;* and (3) is derived from *S. typhimurium*. According to Fig. 6, since the ordinary PCR method shown in Fig. 6(B) amplifies only the sequence defined by the sense primer (SEQ ID NO: 7) and antisense primer (SEQ ID NO: 8), hardly any difference was observed in the waveform patterns of the bacterial strains. In contrast, in the present invention, as shown in Fig. 6(A), multiple regions of a sequence are synthesized, and each of the bacterial strains thus show diverse waveform patterns, enabling identification of the nucleic acids of a bacterial strain by studying these waveform patterns.

After complementary strand synthesis, the nucleotide strands were confirmed by the steps of: adding 2 µL of loading buffer to 10 µL of a sample; electrophoresing the mixture on a 1.2% agarose gel for 45 minutes at 50V; and then staining the gel with ethidium bromide. A 200-bp ladder marker was used as the molecular size marker.

As a comparative example, a similar electrophoresis was performed using 10 µL of a sample which had undergone nucleic acid amplification using the sense primer (SEQ ID NO: 7) and antisense primer (SEQ ID NO: 8), instead of the BAUP65 primer (SEQ ID NO: 6), and the nucleotide strands were confirmed by staining the gel with ethidium bromide.

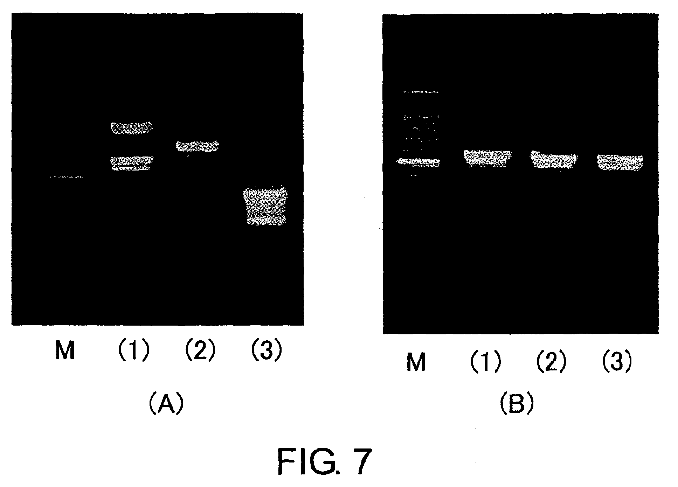

The electrophoretic analysis results of the reaction products from this invention and from the PCR method are shown in Figs. 7(A) and (B), respectively. In Fig. 7, lane 1 is *C*. *jejuni;* lane 2 is *H*. *Influenzae;* and lane 3 is *S*. *typhimurium*. As is apparent from Fig. 7, the ordinary PCR method amplifies only the sequence defined by the sense primer (SEQ ID NO: 7) and antisense primer (SEQ ID NO: 8), and thus only bands of the same size are observed in Fig. 7(B). In contrast, as shown in Fig. 7(A), multiple bands were observed for the present invention. This confirms that the present invention synthesized the sequences of multiple regions.

### (2-2) Comparison of dissociation curves based on annealing temperature

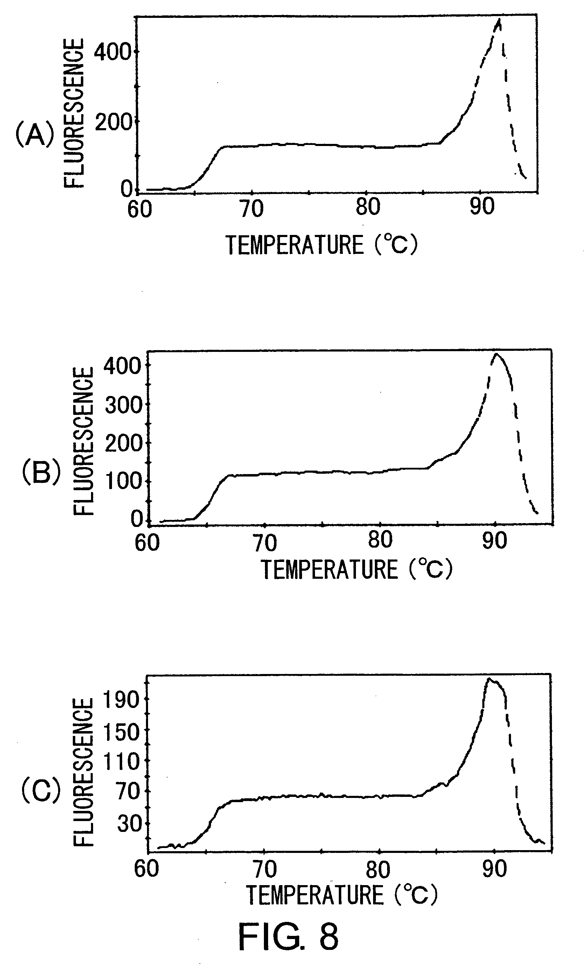

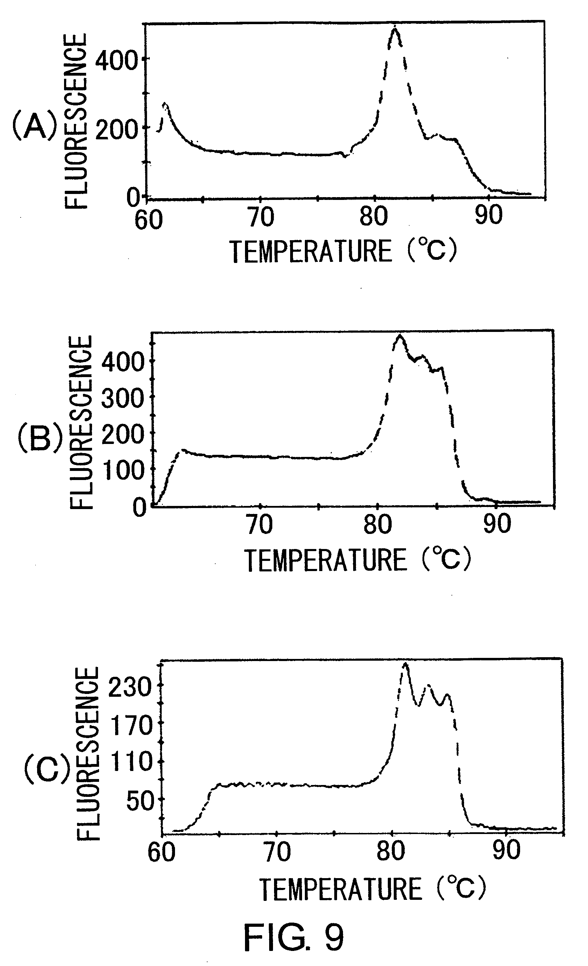

In order to confirm changes in dissociation curve waveform patterns due to differences in annealing temperature, complementary strand synthesis was performed for two strains of bacteria, *Escherichia coli* and *Staphylococcus aureus,* at three temperatures: 55°C, 40°C, and 25°C, using the same methods as described above. The dissociation curve waveforms were observed. The waveform patterns obtained for *E*. *coli* and *S*. *aureus* are shown in Figs. 8 and 9, respectively. Figs. 8 and 9 show that the obtained waveforms became more characteristic for both bacteria with the decrease of annealing temperature from 55°C to 40°C and then to 25°C. This proves that as annealing temperature decreases, the types of primers that initiate complementary strand synthesis increase.

### (2-3) Comparison of dissociation curves based on the presence or absence of an ambiguous region

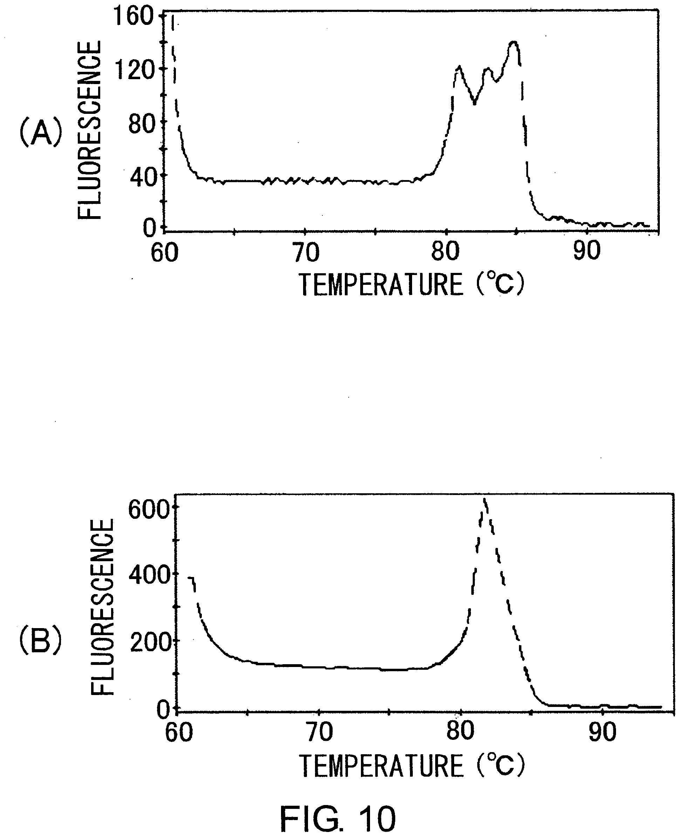

In order to confirm changes in dissociation curve waveform patterns due to the presence or absence of ambiguous regions, complementary strand synthesis was performed on *S. aureus* using BAUP65 primer (SEQ ID NO: 6), and a primer lacking an ambiguous region (5'-GATCCAACCGC-3'/SEQ ID NO: 9), and the dissociation curve waveforms were then observed. Figs. 10(A) and (B) show the waveform patterns obtained when using BAUP65 primer (SEQ ID NO: 6), and the primer lacking the ambiguous region, respectively. As evident from Fig. 10, BAUP65 primer (SEQ ID NO: 6) comprising an ambiguous region results in a more characteristic waveform than a primer lacking an ambiguous region. This indicated that, by using primers comprising an ambiguous region, nucleic acids can be more effectively identified.

### [Example 3] The use of different primer complexes for waveform production

An identification method using a kit of this invention, as shown in Fig. 11, was simulated. An objective of the kit shown in Fig. 11 is to yield more diverse waveform patterns by utilizing primer complexes for producing multiple types of waveforms, where the primer complexes are designed by targeting different regions. This Example used three types of primers for waveform production: sPGBUP65, sPGBUPUPR, and sPGBUPFX. These primer complexes for waveform production were individually designed by selecting different regions as the target region, as shown in Fig. 12.

The same reaction solution composition as in Table 2 was used to perform the complementary strand syntheses, except that each of the primer complexes for waveform production was used instead of BUP65. Complementary strand synthesis was performed by repeating the Example 1 reaction cycle 70 times. DNAs extracted from *E*. *coli, S*. *aureus,* and *B. cereus* were used as test nucleic acids. ISOGEN-LS (Nippon Gene) was used to extract the DNAs.

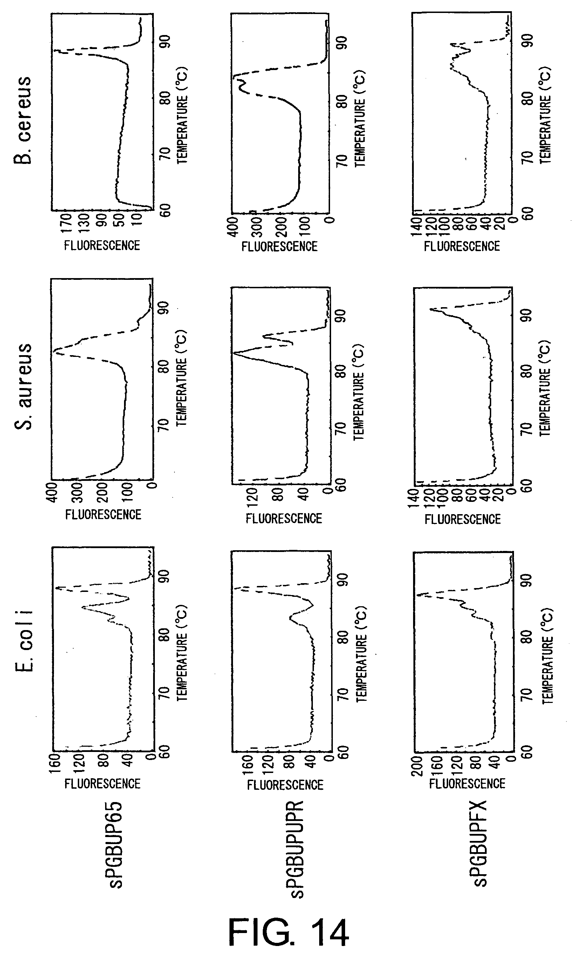

The obtained dissociation curve waveform patterns are shown in Fig. 14. Different waveform patterns were obtained depending on the test nucleic acids and primers used. Specifically, this showed that using different primer complexes for waveform production yields more diverse waveform patterns. By comparing each waveform pattern to a reference waveform pattern, sample DNAs can be identified.

### [Example 4] The effect of a "stabilizer" on low-temperature annealing at 25°C

Hereinafter, the effect of a "stabilizer" in this invention is specifically illustrated with reference to Examples. However, the present invention is not to be construed as being limited to the following Examples, and various modifications are possible within the scope of this invention.

### (1) Method

Primers BAUP65 (SEQ ID NO: 10), which recognize specific nucleotide sequences of bacterial DNA at multiple regions, were produced to identify nucleic acids based on the methods of this invention. In the step of synthesizing a complementary strand, the waveform patterns obtained with or without addition of a stabilizer were compared to confirm the effect of the stabilizer.

*E. coli* DNA was used as the bacterial DNA. The DNA was extracted using ISOGEN-LS (Nippon Gene) from approximately 1x 10⁶ cells of *E. coli* by following the instructions. The extracted DNA was prepared to a final concentration of 0.5 µg/µL (H₂O). The "stabilizer" formulation is shown in Table 3, and the reaction solution compositions for complementary strand synthesis are shown in Tables 4 and 5.

**Table 3.**

| Formulation for 1 mL of "stabilizer" | |
|---|---|
| Formulation of the reaction solution | Formulated amount |
| 0.2 M Na₂SO₄ | 100 µL |
| 1% SDS*¹ | 50 µL |
| 20% Brj35*² | 500 µL |
| Sterilized distilled water | 350 µL |
| Total | 1000 µL |

**Table 4.**

| Composition "with no stabilizer" | |
|---|---|
| Formulation of the reaction solution | Formulated amount |
| Sample DNA solution (comprising 1 µg of DNA) | 2 µL |
| 10x PCR buffer (no MgCl₂) | 5 µL |
| 25 mM MgCl₂ | 3 µL |
| SyGreen (sybergreen) solution*³ | 5 µL |
| dNTP (10 mM each) | 1 µL |
| BAUP65 primers (10 pmol/µL) | 18 µL |
| Taq DNA polymerase (5 U/µL)*⁴ | 0.5 µL |
| Sterilized distilled water | 15.5 µL |
| Total | 50 µL |

**Table 5.**

| Composition "with a stabilizer" | |
|---|---|
| Formulation of the reaction solution | Formulated amount |
| Sample DNA solution (comprising 1 µg of DNA) | 2 µL |
| 10x PCR buffer (no MgCl₂) | 5 µL |
| 25 mM MgCl₂ | 3 µL |
| SyGreen (sybergreen) solution | 5 µL |
| dNTP (10 mM each) | 1 µL |
| BAUP65 primers (10 pmol/µL) | 18 µL |
| Taq DNA polymerase (5 U/µL) | 0.5 µL |
| Sterilized distilled water | 5.5 µL |
| "Stabilizer" | 10 µL |
| Total | 50 µL |
| *1 SDS : Sodium Dodecyl Sulfate | |
| *2 Brj35 : Polyoxyethylene (23) Lauryl Ether | |
| *3 SyGreen solution : 1/1000 dilution of stock solution | |
| *4 Taq DNA polymerase : TAKARA Ex Taq™ R-PCR BAUP65 : 5'-GGAAGGTGGGG-3' | |

Complementary strand synthesis was performed by adding 50 µL of one sample to each of the reaction solutions comprising compositions "with no stabilizer", and compositions "with a stabilizer". The reactions were performed in duplicates, and waveforms from a total of four samples were observed. Complementary strands were synthesized by a protocol using conditions of "50 cycles of 98°C for two seconds, 25°C for 40 seconds, and 72°C for ten seconds" using iCycler (Biorad). After the reaction, the dissociation curve waveforms of the synthesis products were plotted by an eight-second observation at each temperature between 75°C to 95°C, increasing the temperature by 0.1°C.

After the nucleic acid amplification reaction, the synthesis products were confirmed by the steps of: adding 2 µL of loading buffer to 10 µL of sample; electrophoresing the mixture on a 1.2% agarose gel for 45 minutes at 50V; and then staining the gel with ethidium bromide. A 200-bp ladder marker was used as the molecular size marker.

### (2) Results

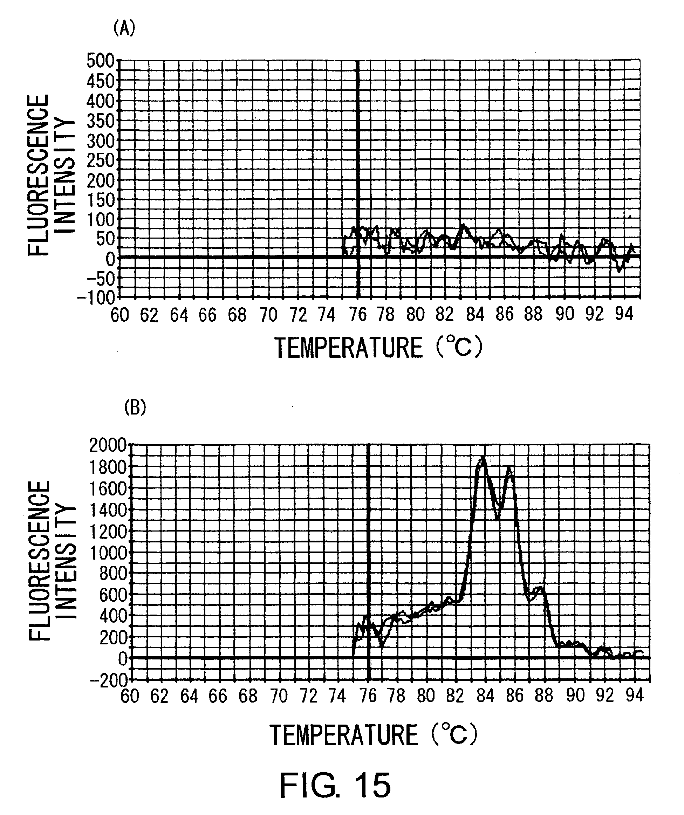

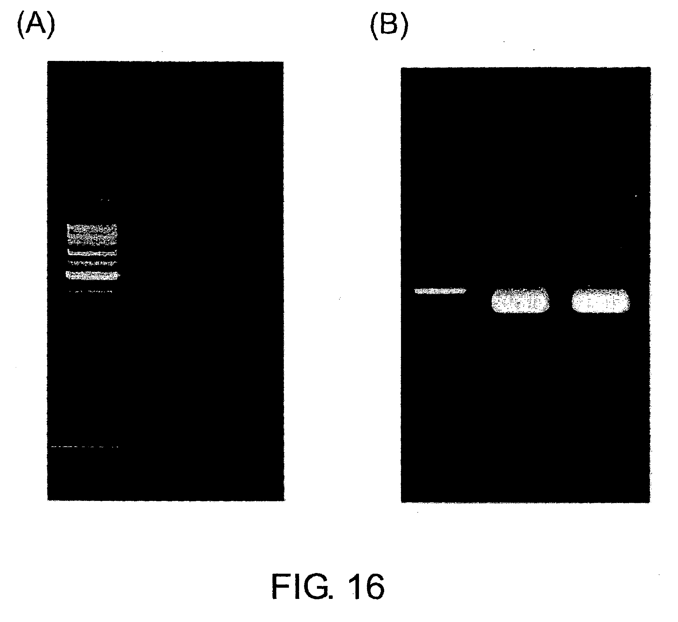

The effect of a "stabilizer" is shown as the difference in the waveforms of Fig. 15 (A) : "with no stabilizer"; and Fig. 15 (B) : "with a stabilizer". In Fig. 15(A), the waveform is ambiguous due to insufficient nucleic acid synthesis. On the other hand, in Fig. 15 (B) , since sufficient amounts of nucleic acids were synthesized, the characteristic waveform of *E. coli* DNA is plotted normally. The effect of the "stabilizer" was verified by the results of electrophoresis analysis. Specifically, in Fig. 16(A), "with no stabilizer", synthesis product bands were not found. On the other hand, in Fig. 16(B) "with a stabilizer", synthesis products bands were confirmed.

The above-described results verified that the addition of a "stabilizer" enables complementary strand synthesis at low temperature conditions of 25°C, enabling waveform analysis. A stabilizer is considered to enable specific annealing by primers and complementary strand synthesis at 25°C through mechanisms such as:
the maintenance of linearization of primers and DNA;
the suppression of higher order structure formation; or
the stabilization of hydrogen bonds between primers and template DNA.

Therefore, the addition of a "stabilizer" is effective when performing this invention by complementary strand synthesis under low temperature conditions.

### Industrial Applicability

The methods for identifying nucleic acids of this invention can detect slight differences in nucleotide sequences in nucleic acids that have a high structural identity, detecting these differences as clear differences in dissociation curve waveform patterns. In the methods for identification of this invention, the elongation products obtained by the synthesis of multiple regions of test nucleic acids are used as objects of analysis. Nucleic acids are identified by comparing the dissociation curves of elongation products. A mixture of elongation products obtained from multiple regions is a mixture of polynucleotides comprising various nucleic acids. In the present invention, nucleic acids are identified by analyzing a mixture of diverse polynucleotides. Comparison to a known nucleic acid can be easily carried out by the steps of: elucidating the dissociation curve waveform pattern for a nucleic acid whose structure is already known, using the same conditions as for the methods for identifying nucleic acids of this invention; and comparing this pattern to the dissociation curve waveform pattern of a test nucleic acid.

The methods for identifying nucleic acids of this invention comprise the steps of directly analyzing and obtaining the dissociation curve waveform patterns for mixtures of elongation products. The step of obtaining elongation products can thus be easily performed. Specifically, by annealing primer complexes to test nucleic acids, and repeating complementary strand syntheses by DNA polymerase, the mixtures of elongation products necessary for the present invention can be synthesized in one reaction vessel.

In contrast, methods for identifying nucleic acids based on known nucleic acid synthesis methods comprise the step of analyzing highly homogeneous synthesis products. For example, PCR typically synthesizes one type of polynucleotide, forming a paired double strand that is completely complementary throughout its full length. Accordingly, even if test nucleic acids comprise partly different sequences, the overall thermodynamic stability becomes nearly the same. Such highly homogeneous polynucleotides only yield dissociation curves with similar single peaks. Therefore, it is difficult to identify the nucleic acids by comparing the dissociation curves.

The present invention provides several methods for obtaining elongation products for the methods for identifying nucleic acids of this invention. All of these methods enable the synthesis of complementary strands of multiple regions necessary for analysis using a few primer types. By utilizing these methods, the number of primers necessary for the reaction can be reduced. Using few primer types for the complementary strand synthesis of multiple regions means that primers for each region can be provided in sufficient amounts with certainty. In addition, it is economically advantageous to use few primer types.

PCR amplifies nucleic acids exponentially, while the primer complexes for waveform production of this invention cannot be expected to yield exponential amplification. However, since multiple regions become synthesis targets, relatively large amounts of polynucleotides are easily synthesized. As a result, polynucleotides necessary for dissociation curve analysis can be synthesized over a short reaction time.

The methods for identifying nucleic acids of this invention are useful, for example, for identifying genetic polymorphisms. Currently, much information is being accumulated on SNPs, which are one type of genetic polymorphism. In order to actually make clinical use of these SNP analysis results, each of the multiple SNPs thought to be related to a disease must be simultaneously identified. However, methods currently used for genotyping comprise a number of issues with their speed and expense to use them in simultaneously analyzing multiple SNPs. On the other hand, by utilizing the methods for identification of this invention, a combination of multiple SNPs may be simultaneously identified by one type (or few types) of primer complex. Using an identification method of this invention, slight differences in the nucleotide sequences of synthesized polynucleotides can be identified as clear differences in dissociation curves. Therefore, differences in multiple SNPs can be found using a single analysis.

All prior art references cited herein are incorporated into this description as references.

## Claims

1. A method for identifying a nucleic acid, wherein the method comprises the steps of:
(1) synthesizing a nucleic acid comprising a nucleotide sequence complementary to multiple regions of a test nucleic acid;
(2) obtaining dissociation curves for a mixture of the nucleic acid synthesized in step (1); and
(3) comparing the waveform patterns of the dissociation curves and identifying nucleic acids comprising the same waveform pattern to have the same nucleotide sequence.

2. The method of claim 1, wherein step (1) of synthesizing a nucleic acid comprising a nucleotide sequence complementary to multiple regions of a test nucleic acid, comprises the step of synthesizing a complementary strand by annealing one or more types of primer comprising a nucleotide sequence complementary to multiple regions of the test nucleic acid.

3. The method of claim 2, which comprises the step of synthesizing the complementary strand by annealing the primers in the presence of a denaturant and/or salt.

4. The method of claim 3, wherein the denaturant is selected from the group consisting of nonionic surfactants, anionic surfactants, and detergents.

5. The method of claim 4, wherein the nonionic surfactant is any one selected from the group consisting of a polyoxyethylene ether of glycerol ester, a polyoxyethylene ether of sorbitan ester, and a polyoxyethylene ether of sorbitol ester.

6. The method of claim 4, wherein the detergent is any compound selected from the group consisting of dodecyl sulfate, lauroylsarcosine salt, laurylate, and mercaptoacetate.

7. The method of claim 3, wherein the salt is any compound selected from the group consisting of Na₂SO₄, Na₂SO₃, NaH₂PO₄, and NaHCO₃.

8. The method of claim 2, wherein the primers comprise one type of oligonucleotide that can anneal to multiple regions of the test nucleic acid.

9. The method of claim 2, wherein the primers comprise two or more types of oligonucleotides that can anneal to multiple regions of the test nucleic acid.

10. The method of claim 9, wherein the nucleotide sequences of the multiple regions are partially identical.

11. The method of claim 10, wherein the nucleotide sequences of the primers are partially different, and wherein the different nucleotides are at any position on the primer nucleotide sequence.

12. The method of claim 10, wherein the nucleotide sequences of the primers are partially different, and wherein the different nucleotides are localized to the 5'-side of the primer nucleotide sequence.

13. The method of claim 12, wherein the primers constitute a primer complex for producing waveforms, wherein the primer complex comprises:
a specific primer, which comprises a nucleotide sequence complementary to a target region of a template nucleic acid; and
at least one type of ambiguous primer, which comprises the following specific region and ambiguous region:
a specific region, which comprises the 3' end of the oligonucleotide and consists of a nucleotide sequence complementary to the target region; and
an ambiguous region, which is positioned to the 5' side of the specific region, and comprises a nucleotide sequence wherein a nucleotide comprised in the nucleotide sequence complementary to the target region is substituted with a nucleotide other than the nucleotide.

14. A method for identifying a nucleic acid, wherein the method comprises the steps of:
selecting multiple regions as target regions in a template nucleic acid; and
performing the method of claim 13 on a single test nucleic acid using the multiple target regions as objects of analysis.

15. The method of claim 13, which comprises the step of:
annealing the primer complex to the template nucleic acid at a temperature that is 20°C to 40°C lower than the melting temperature of the specific primer.

16. The method of claim 13, wherein multiple cycles of the primer complex annealing step and the complementary strand synthesis step are performed.

17. The method of claim 1, wherein the test nucleic acid is single stranded or double stranded.

18. The method of claim 1, wherein the test nucleic acid is a DNA or RNA.

19. The method of claim 1, wherein the test nucleic acid is a genomic DNA, and wherein step (1) comprises the step of synthesizing at least one region whose nucleotide sequence in a cell to be identified differs from that in an another cell.

20. The method of claim 19, wherein the nucleotide sequences of the primers for synthesizing multiple regions are at least partially identical.

21. The method of claim 19, wherein the test nucleic acid is a genomic DNA of a microorganism, and wherein step (1) comprises the step of synthesizing at least one region whose nucleotide sequence in the microorganism to be identified differs from that in an another microorganism.

22. The method of claim 19, wherein the test nucleic acid is a genomic DNA of a eukaryotic cell, and wherein step (1) comprises the step of synthesizing a region comprising a group of genes in which a nucleotide sequence is conserved.

23. The method of claim 22, wherein the method comprises the step of synthesizing multiple regions using a primer comprising a nucleotide sequence complementary to a nucleotide sequence that is conserved among the genes.

24. A method for producing dissociation curve waveform patterns as a reference for identifying a nucleic acid, wherein the method comprises the steps of:
(1) synthesizing nucleic acids, which comprise nucleotide sequences complementary to multiple regions of a standard nucleic acid sample, as test nucleic acids; and
(2) obtaining dissociation curves for the mixture of nucleic acids synthesized in step (1).

25. A method for producing a reference dissociation curve waveform pattern for multiple types of standard nucleic acid samples by using the method of claim 24.

26. The method of claim 25, wherein the method comprises the step of obtaining a dissociation curve for multiple types of standard nucleic acid samples that are synthesized by a common primer complex.

27. A reference dissociation curve waveform pattern database that comprises multiple reference dissociation curve waveform patterns, obtained by the method of claim 24.

28. A primer complex for waveform production, which comprises a mixture of one or more types of primers whose nucleotide sequences are complementary to multiple regions in a test nucleic acid.

29. The primer complex of claim 28, wherein the primer is one type of oligonucleotide that can anneal to multiple regions in the test nucleic acid.

30. The primer complex of claim 28, wherein the primers are two or more types of oligonucleotides that can anneal to multiple regions in the test nucleic acid.

31. The primer complex of claim 30, wherein the nucleotide sequences of the multiple regions are partially identical.

32. The primer complex of claim 30, wherein the nucleotide sequences of the primers are partially different, and wherein the different nucleotides are at any position in the primer nucleotide sequence.

33. The primer complex of claim 30, wherein the nucleotide sequences of the primers are partially different, and wherein the different nucleotides are localized to the 5' side of the primer nucleotide sequence.

34. The primer complex of claim 33, that comprises:
a specific primer, which comprises a nucleotide sequence complementary to a target region of a template nucleic acid; and
at least one type of ambiguous primer, which comprises the following specific region and ambiguous region:
a specific region, which comprises the 3' end of a primer and consists of a nucleotide sequence complementary to the target region; and
an ambiguous region, which is positioned to the 5' side of the specific region, and comprises a nucleotide sequence wherein a nucleotide comprised in the nucleotide sequence complementary to the target region is substituted with a nucleotide other than the nucleotide.

35. The primer complex of claim 34, wherein the gc content in the specific region of each primer is 50% or more.

36. The primer complex of claim 34, wherein the primer complex is used for waveform production and wherein the variety of the substituted nucleotide in the ambiguous region of the ambiguous primer increases from the 3' side to the 5' side.

37. The primer complex of claim 34, wherein the nucleotide sequence of the ambiguous region comprises the following three regions, and wherein the primer complex comprises ambiguous primer complexes having all combinations of substituted nucleotide sequences that constitute each of the three regions,
(1) an N region constituting the 5'-terminus of the ambiguous region, in which each of the nucleotides of its nucleotide sequence are substituted with random three types of nucleotides other than the nucleotide complementary to a target region nucleotide, wherein the three types of nucleotides are selected from adenine, cytosine, guanine, and thymine,
(2) a 3 ambiguous region, positioned to the 3'-side of the N region, in which each of the nucleotides of its nucleotide sequence are substituted with random two types of nucleotides other than the nucleotide complementary to a target region nucleotide, wherein the three types of nucleotides are selected from adenine, cytosine, guanine, and thymine, and
(3) a 2 ambiguous region, positioned to the 3'-side of the 3 ambiguous region, in which each of the nucleotides of its nucleotide sequence are substituted with a random type of nucleotide other than the nucleotide complementary to a target region nucleotide, wherein the random type of nucleotide is selected from adenine, cytosine, guanine, and thymine.

38. The primer complex of claim 37, wherein the number of nucleotides of the N region of the primer is two to four.

39. The primer complex of claim 37, wherein the ratio of the number of nucleotides of the N region: 3 ambiguous region: 2 ambiguous region of the primer is 1:2:1.

40. The primer complex of claim 34, wherein the number of nucleotides of an ambiguous region of an ambiguous primer of the primer complex is 10% to 80% of the number of nucleotides of the primer.

41. The primer complex of claim 34, wherein the total number of nucleotides in the specific region and ambiguous region of the ambiguous primers of the primer complex is ten to 30 nucleotides.

42. A method for producing a primer complex for waveform production,
wherein the complex comprises:
a specific primer, which comprises a nucleotide sequence complementary to a target region of a template nucleic acid; and
at least one type of ambiguous primer, which comprise the following specific region and ambiguous region:
a specific region, which comprises the 3' end of a primer and consists of a nucleotide sequence complementary to the target region; and
an ambiguous region, which is positioned to the 5' side of the specific region, and comprises a nucleotide sequence wherein the nucleotide comprised in the nucleotide sequence complementary to the target region is substituted with a nucleotide other than the nucleotide; and
wherein the method comprises the steps of:
a) synthesizing the specific region; and
b) synthesizing the ambiguous region by binding a nucleotide of a nucleotide sequence complementary to the target region, to a mixture of random nucleotides other than said nucleotide, selected from adenine, cytosine, guanine, and thymine.

43. The method of claim 42, wherein the number of random nucleotides increases from one to three starting from the 3' side to the 5' side of the ambiguous region.

44. A kit for identifying a nucleic acid, wherein the kit comprises the components of:
(1) a primer complex for waveform production, that comprises a mixture of one or more types of primers comprising nucleotide sequences complementary to multiple regions of a test nucleic acid;
(2) a DNA polymerase that catalyzes template-specific synthesis of a complementary strand; and
(3) a substrate for complementary strand synthesis.

45. The kit of claim 44, wherein the primers are one type of oligonucleotide that can anneal to multiple regions of the test nucleic acid.

46. The kit of claim 44, wherein the primers are two or more types of oligonucleotides that can anneal to multiple regions of the test nucleic acid.

47. The kit of claim 46, wherein the nucleotide sequences of multiple regions are partially identical.

48. The kit of claim 47, wherein the nucleotide sequences of the primers are partially different, and wherein the different nucleotides are at any position in the primer nucleotide sequence.

49. The kit of claim 47, wherein the nucleotide sequences of the primers are partially different, and wherein the different nucleotides are localized to the 5' side of the nucleotide sequences of the primers.

50. The kit of claim 49, wherein the primer complex comprises a complex of:
a specific primer, which comprises a nucleotide sequence complementary to the target region of a template nucleic acid; and
at least one type of ambiguous primer, which comprises the following specific region and ambiguous region:
a specific region, which comprises the 3' end of a primer and consists of a nucleotide sequence complementary to the target region; and
an ambiguous region, which is positioned to the 5' side of the specific region, and comprises a nucleotide sequence wherein a nucleotide comprised in the nucleotide sequence complementary to the target region is substituted with a nucleotide other than the nucleotide.

51. The kit of claim 49, which comprises multiple target regions.

52. The kit of claim 50, wherein the primer complex for waveform production for multiple regions is pre-loaded into individual reaction vessels.

53. The kit of claim 49, which further comprises the dissociation curve waveform patterns of a positive control and/or nucleic acid to be identified.

54. The kit of claim 44, which further comprises a denaturant and/or salt.

55. The kit of claim 54, wherein the denaturant is selected from the group consisting of nonionic surfactants, anionic surfactants, and washing agents.

56. The kit of claim 55, wherein the nonionic surfactant is selected from the group consisting of a polyoxyethylene ether of glycerol ester, a polyoxyethylene ether of sorbitan ester, and a polyoxyethylene ether of sorbitol ester.

57. The kit of claim 55, wherein the detergent is any compound selected from the group consisting of dodecyl sulfate, lauroylsarcosine salt, laurylate, and mercaptoacetate.

58. The kit of claim 54, wherein the salt is any compound selected from the group consisting of Na₂SO₄, Na₂SO₃, NaH₂PO₄, and NaHCO₃.
